(19) Europäisches Patentamt
European Patent Office
Office européen des brevets

(11) EP 2 260 029 B1

(12) **EUROPEAN PATENT SPECIFICATION**

(45) Date of publication and mention of the grant of the patent:
**01.10.2014 Bulletin 2014/40**

(21) Application number: **09715018.9**

(22) Date of filing: **20.02.2009**

(51) Int Cl.:
***C07D 265/32*** *(2006.01)* ***C07F 9/6518*** *(2006.01)*

(86) International application number:
**PCT/EP2009/052036**

(87) International publication number:
**WO 2009/106486 (03.09.2009 Gazette 2009/36)**

(54) **PREPARATION OF MORPHOLINE DERIVATIVES**

HERSTELLUNG VON MORPHOLINDERIVATEN

PRÉPARATION DE DÉRIVÉS DE MORPHOLINE

(84) Designated Contracting States:
**AT BE BG CH CY CZ DE DK EE ES FI FR GB GR HR HU IE IS IT LI LT LU LV MC MK MT NL NO PL PT RO SE SI SK TR**

(30) Priority: **26.02.2008 EP 08151949**

(43) Date of publication of application:
**15.12.2010 Bulletin 2010/50**

(73) Proprietor: **Sandoz AG**
**4056 Basel (CH)**

(72) Inventors:
- **ALBERT, Martin**
  **6250 Kundl/Tirol (AT)**
- **DE SOUZA, Dominic**
  **6250 Kundl/Tirol (AT)**
- **KNEPPER, Kerstin**
  **6250 Kundl/Tirol (AT)**

(74) Representative: **Wichmann, Hendrik et al**
**Wuesthoff & Wuesthoff**
**Schweigerstrasse 2**
**81541 München (DE)**

(56) References cited:
**WO-A-2007/044829 US-A1- 2007 265 442**

- **P. J. PYE ET AL: "Crystallization-induced diastereoselection: asymmetric synthesis of substance P inhibitors" CHEMISTRY - A EUROPEAN JOURNAL., vol. 8, no. 6, 2002, pages 1372-1376, XP002494210 USVCH PUBLISHERS.**
- **M. M. THAO ET AL: "Practical Asymmetric Synthesis of Aprepitant, a Potent Human NK-1 Receptor Antagonist, via a Stereoselective Lewis Acid-Catalyzed Trans Acetalization Reaction" JOURNAL OF ORGANIC CHEMISTRY., vol. 67, no. 19, 2002, pages 6743-7647, XP002494211 USAMERICAN CHEMICAL SOCIETY, WASHINGTON, DC. cited in the application**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 260 029 B1

## Description

### Field of invention

This invention relates to processes and intermediates for the stereoselective synthesis of substituted morpholine derivatives, which can serve as $NK_1$ receptor antagonists. In particular, the invention allows the stereoselective preparation of new compounds, which allow an efficient access to aprepitant and fosaprepitant, two potent and orally active $NK_1$ antagonists.

### Background of the invention

Aprepitant (compound I; figure 1) has been first disclosed in EP 0734381 B1 and is currently being marketed as a treatment for chemotherapy-induced nausea and vomiting under the trade name Emend. In EP 0734381 B1 a synthetic route for this compound and a series of other morpholine derivatives is described. However, the disclosed processes for these compounds suffer from lengthy syntheses and low efficiency, which limits their use on industrial scale.

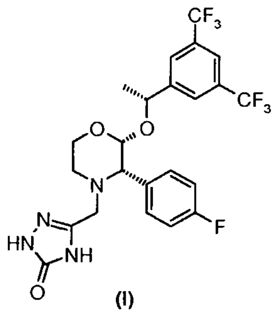
(I)
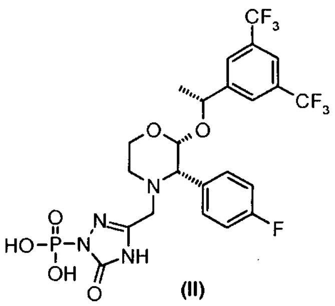
(II)
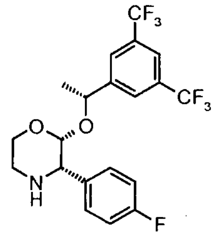
(III)

**Figure 1.** Structure of aprepitant (**I**), fosaprepitant (**II**), and the key intermediate (**III**) for the synthesis of these compounds

In EP 0748320 B1 the structure of fosaprepitant (compound **II,** figure 1) is disclosed. The preparation is based on an appropriate phosporyl transfer to aprepitant, optionally followed by a removal of phosphoryl protecting groups. In WO 99065900 A1 an improved process for the introduction of the 1,2,4-triazolin-5-on-yl-methyl side chain by using 3-chloromethyl-1,2,4-triazolin-5-one as alkylating agent is described. The novel process enables a one pot - one step process for the introduction of the side chain, thereby improving the prior art one pot - two step process. In WO 2003089429 A1 the prior art process for the introduction of the side chain is improved by conducting step 2 of the introduction of the side chain at a temperature ranging from 140°C to 150°C.

An improved stereoselective synthesis for the key intermediate of formula III (figure 1) is disclosed in WO2001096319 A1. The process makes use of a chiral auxiliary attached to the nitrogen of the morpholine ring. A major drawback of this route is that the chiral auxiliary is destroyed during removal thus making the process expensive.

Another synthesis of key intermediate of formula **III** is described in WO 2001096320 A1. This route makes use of an intramolecular rearrangement to correctly set the hard to make acetal center. The stereochemistry of the exocyclic methyl group is established by a prior art process, giving an unfavorable mixture of isomers at this center.

US 5668280 A and US 6130331 A disclose approaches for the synthesis of key intermediate of formula **III** based on crystallization induced diastereomeric transformations. In both patents a selective reduction of a lacton using an expensive metal hydride is required.

In WO2007044829 A2 a mixture of 4 of 8 possible stereoisomers is prepared in an unselective manner. The desired isomer is isolated by several purification steps from this mixture of isomers, resulting in a low overall yield.

In WO 2001094322 A1, WO 2001094323 A1, and WO 2001094324 A1 an efficient process for the synthesis of the key intermediate of formula III by making use of a crystallization induced diastereomeric transformation is described. In this process the isolation of only three intermediates en route to aprepitant is required, making this process interesting from an economical point of view.

Chemistry- A european Journal 8(6),2002,1372-76 describes a stereoselective process which uses a chiral auxiliary attached to the nitrogen for the preparation of similar compounds.

Although some efficient processes for the synthesis of aprepitant and fosaprepitant are available, further improvements in terms of number of isolated intermediates and overall yield would be highly desirable.

In the following such an improved process is described. We have found, that a very efficient three component coupling, which allows the construction of the morpholine core in one step, can be combined with a selective crystallization

with a chiral acid, which allows to establish the required stereochemistry. The chiral acid can easily be recovered and reused; the undesired isomers can be recycled by racemization.

En route to aprepitant only two intermediates need to be isolated, making the described process highly economical.

## The invention

The present invention relates to a process for the asymmetric synthesis of aprepitant or fosaprepitant, comprising the steps of

a) a three component coupling of an amino alcohol of formula VI, of 4-fluorophenylboronic acid or a $C_{1-6}$ alkyl or cyclic ester thereof (formula V), and of glyoxal (IV). Crystallization of the obtained morpholine derivative as addition salt with a chiral acid; isolation of a product of formula VII.chiral acid. Optionally, the undesired isomers in the mother liquor are racemized by treatment with acid or base, optionally at higher temperature, and the racemic morpholine derivative is resubjected to a crystallization with a chiral acid.
Optionally, the racemization of the undesired isomers and the crystallization of the desired isomer as a chiral acid addition salt is performed in a one pot fashion;

b) generation of hemi-acetal **VII** free base by portioning compound of formula VII.chiral acid between an alkaline aqueous layer and a water-immiscible organic phase; activation of the hemi-acetal functionality by transforming the OH-group into an activated derivative; reaction of the resulting activated acetal with alcohol of formula **VIII.** Removal of impurities by extraction to get a solution of compound **IX**;

c) removal of the N-protecting group to give the amine of formula **X;**

d) oxidation of the amine of formula **X** to the corresponding cyclic imine of formula **XI**; isolation of the cyclic imine of formula **XI**;

e) reduction of imine **XI** with a catalyst and $H_2$ or an $H_2$ equivalent; removal of the catalyst by filtration to get the key intermediate of formula **III**;

f) alkylation of compound of formula **III** to give aprepitant or fosaprepitant directly or *via* e.g. protected intermediates;

g) optionally, conversion of aprepitant to fosaprepitant by phosphorylation or a phosphorylation - deprotection sequence.

Scheme 1 exemplifies the process of the present invention by way of a sequence of isolated and non-isolated intermediates. Optionally, more intermediates can be isolated. One aspect of this invention is, that it is possible to remove undesired diasteoreomers by isolating the compounds of formula **VII** and **XI.** These compounds are particularly suited for this purpose (good depletion of undesired compounds during crystallization) resulting in a **I** or **II** with high purity.

The process of the invention has the advantage that it is fast, economic, simple, and produces aprepitant and fosaprepitant, respectively, in high yield and high optical purity.

The invention further relates to the new compounds of formula **VII** in crystalline form and to diastereomers of formula **XI, I,** and **II.**

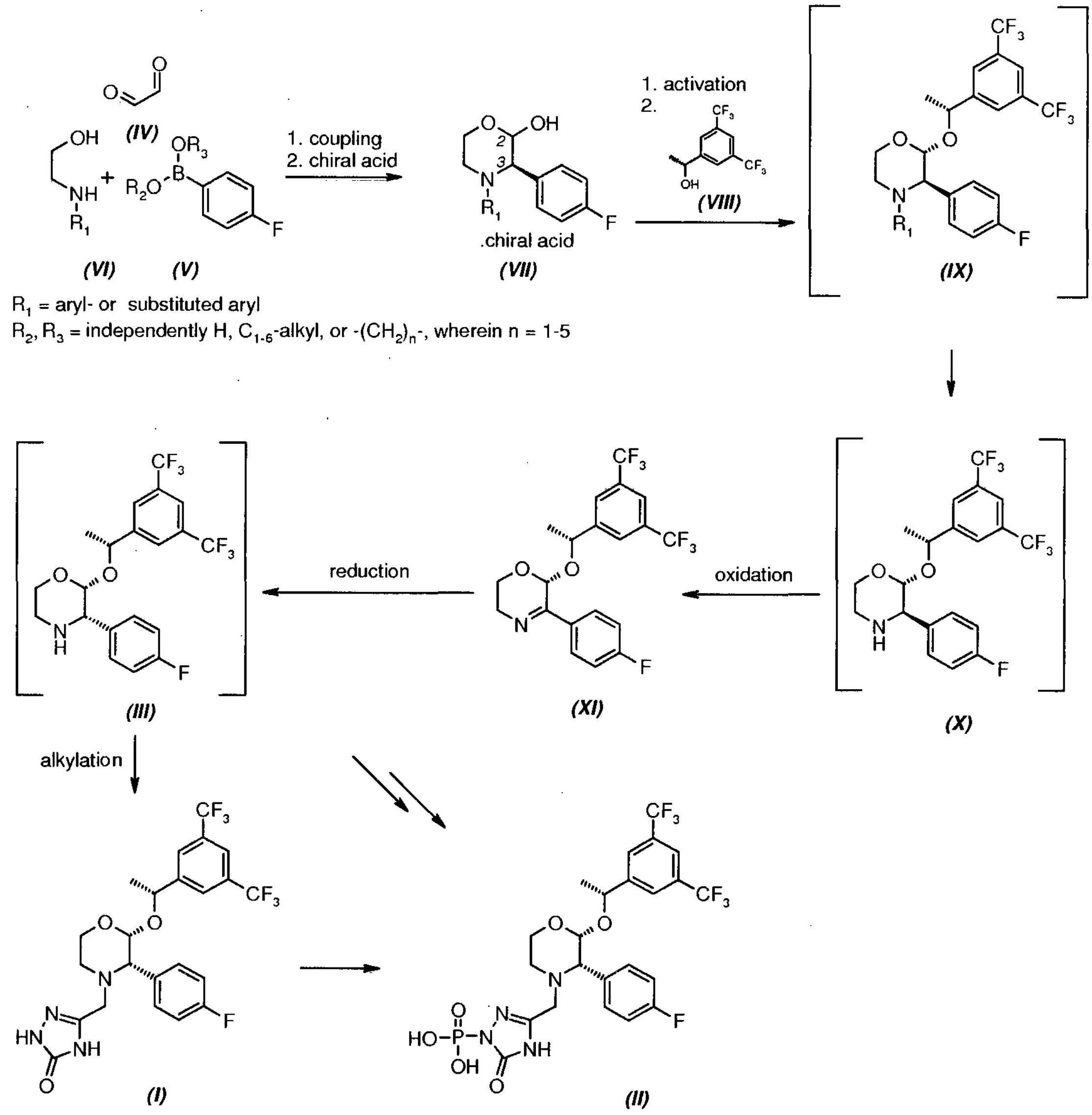


**Scheme 1.** Process for the synthesis of aprepitant or fosaprepitant

Alternatively, a compound of formula iso-**VII** may be isolated as an addition salt with a chiral acid and, following the same reaction sequence as for the preparation of aprepitant or fosaprepitant, the synthesis of isomers of aprepitant or fosaprepitant, usually present as impurities in the synthesis of aprepitant or fosaprepitant is also available.

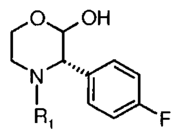

.chiral acid

**iso-VII**

## Detailed description of the invention

The present invention relates to a process for the asymmetric synthesis of morpholine derivatives, preferably to aprepitant and fosaprepitant comprising the steps of

**a)** a three component coupling of an amino alcohol of formula **VI**, of 4-fluorophenylboronic acid or a $C_{1-6}$ alkyl or cyclic ester thereof (formula **V**), and of glyoxal (**IV**). Crystallization of the obtained morpholine derivative as addition salt with a chiral acid; isolation of a product of formula **VII**.chiral acid. Optionally, the undesired isomers in the mother liquor are racemized by treatment with acid or base, optionally at higher temperature, and the racemic morpholine derivative is resubjected to a crystallization with a chiral acid. Optionally, the racemization of the undesired isomers and the crystallization of the desired isomer as a chiral acid addition salt is performed in a one pot fashion;

Preferred protecting groups ($R_1$) for the amino alcohol of formula **VI** are benzyl or substituted benzyl. Most preferably, $R_1$ is benzyl. Instead of benzyl, other nitrogen protecting groups, which are known to the skilled person (see e.g. Theodora W. Greene, Peter G. M. Wuts, Protecting Groups in Organic Syntheses, 3rd ed., 1999, John Wiley & Sons) can be used.

If nitrogen protecting groups other then benzyl or derivatives thereof are used, step c) from the invention has to be modified accordingly (for removal of such protecting groups see Theodora W. Greene, Peter G. M. Wuts, Protecting Groups in Organic Syntheses, 3rd ed., 1999, John Wiley & Sons).

Preferably, $R_2$ and $R_3$ are the same or different and are independently chosen from hydrogen, substituted or unsubstituted $C_{1-6}$ alkyl, substituted or unsubstituted $C_{1-0}$ aralkyl, or form a ring $-(CH_2)_n-$, wherein n = 1-5. Most preferably, $R_2$ and $R_3$ are hydrogen.

Preferred chiral acids are tartaric acid or tartaric acid derivatives such as di-*O,O*'-toluoyl tartaric acid, di-*O,O*'-benzoyl tartaric acid, di-*O,O*'-anisoyl tartaric acid, or *O,O*'-dibenzoyl tartaric acid mono(dimethylamide), camphorsulfonic acid derivatives such as 3-bromocamphor-10-sulfonic acid, camphanic acid, 10-camphorsulfonic, or camphoric acid, amino acids such as glutamic acid, valine, or aspartic acid, mandelic acid or mandelic acid derivatives such as α-methoxy-α-trifluoromethylphenylacetic or α-methoxyphenylacetic acid, acetoxy-5-etienic acid, malic acid, menthyloxyacetic acid, N-(α-methylbenzyl)succinamidic acid, *N*-[1-(1-naphthyl)ethyl]succinamic acid, *N*-(1-phenylethyl)succinamic acid, 1-mono-menthyl phthalate, *N,N*-Bis[1-phenylethyl]phthalamic acid, *N*-(1-phenylethyl)phthalamic acid, 2-phenylpropionic acid, phenylcarbamoyloxypropionic acid, pyroglutamic acid, quinic acid, 1,4-benzodioxane-2-carboxylic acid, 1,1'-binaphthalene-2,2'-diyl hydrogen phosphate, or 5-oxo-2-tetrahydrofurancarboxylic acid in either enantiomeric or diastereomeric form.

However, the invention is not limited to these chiral acids.

Most preferably, the chiral acid is di-*O,O*'-toluoyl-L-tartaric acid.

The amino alcohol **VI**, glyoxal (**IV**), and the phenylboronic acid **V** are dissolved in an organic solvent in the presence or absence of water or in a mixture of organic solvents with or without water. The order of addition is not critical. The reagents can be added in any order. In all cases, compound of formula **VII** in the form of its free base is formed.

Suitable organic solvents are e.g. alcohols, such as ethanol, *n*-butanol, *sec*-butanol, *tert*-butanol, methanol, 2-propanol, or toluene, tetrahydrofuran, acetonitrile, DMF, DMSO, dioxane, DME, diglyme, nitromethane, methyl *tert*-butyl ether, $CH_2Cl_2$, or NMP or mixtures thereof, with toluene and ethanol, 2-propanol, *n*-butanol, *sec*-butanol, or *tert*-butanol, 2-butanol being particularly preferred.

The three-component coupling of compound **VI**, glyoxal, and phenylboronic acid of formula **V** is performed with 0.5 to 1.5 equivalents of boronic acid derivative of formula V relative to amino alcohol of formula **VI,** more preferably with 0.9 to 1.1 equivalents and with 0.8 to 1.5 equivalents of glyoxal relative to amino alcohol of formula **VI,** more preferably with 1.1 to 1.3 equivalents.

The three-component coupling is performed at a temperature between -20°C and 100°C, more preferably between 20°C and 50°C, most preferably at 25°C.

The conversion of amino alcohol of formula **VI** to the product (compound **VII**) is greater than 50%, usually greater than 95%, more preferably greater than 98%.

The isolation of the product of formula **VII** from the reaction mixture can be performed according to methods known to a person skilled in the art. Such methods include extraction, distillation, crystallization, or chromatography.

In a preferred embodiment of the invention, compound **VII** is isolated as an addition salt with a chiral acid. By correct choice of the chiral acid, only one out of four diastereoisomers crystallizes. The solvent of this crystallization is same or different to the solvent of the three component coupling. The solvent in which the crystallization is carried out can be chosen from protic or aprotic solvents or mixtures thereof. Typical solvents are alcohols such as ethanol, 2-propanol, 2-butanol, or *n*-butanol. Optionally the alcohol can be mixed with water or an apolar solvent such as toluene or heptane. However, the invention is not limited to these combinations. In a preferred embodiment, the crystallization is carried out in an alcohol with or without cosolvent. Most preferably the crystallization is carried out in mixtures of 2-propanol and water.

The crystallization is started at elevated temperature and cooling is performed either gradually or using a cooling ramp. The temperature of the crystallization depends on the solvents in use. The start of the crystallization can be at reflux temperature or below.

In a preferred embodiment, the crystallization is carried out in an alcohol and the initial temperature is between

30°C and 100°C, more preferably, between 40°C and 50°C and the reaction mixture is gradually cooled to below 30°C, more preferably, to 0°C to 10°C.

The crystallization can be carried out using 0.4 to 2.0 acid equivalents. This means that 0.4 to 2.0 equivalents of a carboxylic acid with one carboxyl group, 0.2 to 1.0 equivalents of a carboxylic acid with two carboxyl groups, etc. can be used.

A characteristic of the crystallization according to the invention is that a considerably larger amount of one enantiomer out of four possible diastereoisomers of the product **VII** crystallizes as addition salt with the chiral acid. Compound **VII**.chiral acid is typically obtained with an enantiomeric excess (ee) of >50%. In a preferred embodiment the ee is greater 90%. Enantiomeric excess refers to the ratio of diastereoisomers with 3-R configuration to diastereoisomers with 3-S configuration.

The optical purity of salt **VII** obtained after isolation may be improved before further processing. Improvement of the optical purity may be achieved e.g. by recrystallization.

The crystalline product **VII**.chiral acid is isolated by filtration.

Optionally, the mother liquor is heated to such a temperature that racemization of the undesired 3-epimer occurs. A preferred temperature for the racemization is 70 - 100°C. Cooling of the (now racemic) mixture leads to precipitation of the desired crystalline product (**VII**.chiral acid). The process can be repeated several times. Optionally, the reaction mass is concentrated between the individual crystallization / racemization cycles. Optionally an acid, such as HCl or $H_2SO_4$, is added which facilitates the razemisation.

In another embodiment of the invention, the mother liquor is treated with base and compound VII free base is extracted into an organic layer. Compound **VII** free base is then submitted to a racemization by addition of a base or addition of an acid or by stirring at elevated temperature or by a combination of two of these measures. Preferred bases are NaOH or other metal hydroxides. Racemic compound **VII** is then crystallized with a chiral acid as described above.

In another embodiment, crystallization and racemization are performed in a one pot fashion. This is done by carrying out the crystallization under conditions where the desired isomer cyrstallizes while the remaining isomers in the mother liquor undergo racemization;

**b)** generation of hemi-acetal **VII** free base by portioning compound of formula **VII**.chiral acid between an alkaline aqueous layer and a water-immiscible organic phase; activation of the hemi-acetal functionality by transforming the OH-group in an activated derivative; reaction of the activated acetal with alcohol of formula **VIII.** Removal of impurities by extraction to get a solution of compound **IX;**

The conversion of compound **VII** to aprepitant can be performed according to e.g. Zhao, M. M.; McNamara, J. M.; Ho, G.-J.; Emerson, K. M.; Song, Z. J.; Tschaen, D.M.; Brands, K. M. J.; Dolling, U.-H.; Grabowski, E. J. J.; Reider, P.J., J. Org. Chem. 2002, 67, 6743-6747 or WO2001096319 A1.

Surprisingly, we found that the isolation of certain intermediates can be omitted without loss in quality of aprepitant. In particular the isolation of **IX, X,** and **III** can be omitted, thereby resulting in a highly economical process. Most preferably, the synthesis of aprepitant starting from **VII** free base is carried out by isolating only compound **XI.** In another preferred embodiment of the invention, only intermediate **III** or intermediate **X** is isolated en route to aprepitant starting from **VII** free base. In other embodiments of the invention, other intermediates are additionally isolated.

For the conversion of **VII**.chiral acid to **VII** free base, **VII**.chiral acid is suspendend in a mixture of water and of a water-immiscible organic solvent. Addition of base, preferably aqueous NaOH, $NaHCO_3$, or $Na_2CO_3$ generates compound **VII** free base which is extracted into the organic layer.

The organic layer can be dried by e.g. azeotropic distillation or addition of a drying agent, which is removed prior to further processing. Compound **VII** free base is then activated for coupling with alcohol **VIII.** In a preferred embodiment, the activation is carried out by treatment of **VII** free base with a base, preferably $K_2CO_3$, and $Cl_3CCN$, or a base and trifluoroacetic acid anhydride. Other activation methods which are known to a person skilled in the art can be applied. After activation, coupling is performed with 1.0 to 2.0 equivalents of alcohol **VIII.** Activation and coupling of compound **VII** free base or diastereoisomers and derivatives thereof with alcohol **VIII** are described in Zhao, M. M.; McNamara, J. M.; Ho, G.-J.; Emerson, K. M.; Song, Z. J.; Tschaen, D.M.; Brands, K. M. J.; Dolling, U.-H.; Grabowski, E. J. J.; Reider, P.J., J. Org. Chem. 2002, 67, 6743-6747. The conditions used in this publication can be applied to the described process.

In a preferred embodiment compound **IX** is further processed in solution after work-up without isolation. Work-up includes washing of the reaction mass with aqueous base, or aqueous acid, or both consecutively and in any order. Optionally, compound **IX** is isolated by crystallization;

c) removal of the N-protecting group to give the amine of formula **X;**

The removal of the protecting group depends on the nature of the protecting group and can be perfomed with

methods known to a person skilled in the art. Such protecting groups and methods for their removal are described in Theodora W. Greene, Peter G. M. Wuts, Protecting Groups in Organic Syntheses, 3rd ed., 1999, John Wiley & Sons.

In a preferred embodiment of the invention, $R_1$ = benzyl or substituted benzyl and the protecting group is removed by hydrogenolysis. Such a conversion is described in Zhao, M. M.; McNamara, J. M.; Ho, G.-J.; Emerson, K. M.; Song, Z. J.; Tschaen, D. M.; Brands, K. M. J.; Dolling, U.-H.; Grabowski, E. J. J.; Reider, P.J., J. Org. Chem. 2002, 67, 6743-6747. If $R_1$ = benzyl, the protecting group is removed by hydrogenolysis using $H_2$ or a hydrogen donor in the presence of a catalyst such as Pd.

After removal of the protecting group a compound of formula **X** is obtained. In a preferred embodiment, compound **X** is further processed in solution after work-up without isolation. Optionally, compound **X** is isolated by crystallization. Work-up includes optionally filtration of a catalysts and washing of the reaction mass with aqueous base, or aqueous acid, or both consecutively and in any order;

> **d)** oxidation of the amine of formula **X** to the corresponding cyclic imine of formula **XI**; isolation of the cyclic imine of formula **XI;**

Suitable systems for such oxidations are combinations of an oxidizing agent and a base. In a preferred embodiment, *N*-chlorosuccinimid or *N*-bromosuccinimid in combination with DBU are used as reagents and the reaction is carried out in DMF as solvent. Another preferred oxidation is based on the use of NaOCl as oxidizing agent.

After the reaction and aqueous work-up, the product of formula **XI** is isolated by crystallization. Preferably, compound **XI** is crystallized from an alcohol, or a mixture of an alcohol and water or an organic cosolvent. Imin **XI** is obtained in high purity; the level of diastereoisomer iso-**XI** is below 5%, preferentially below 1%.

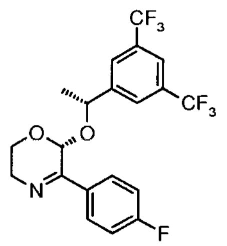

***(XI)***

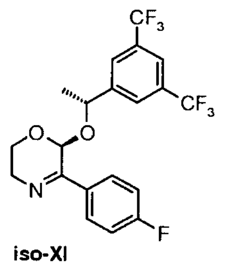

**iso-XI**

> **e)** reduction of imine **XI** with a catalyst and $H_2$ or an $H_2$ equivalent; removal of the catalyst by filtration to get the key intermediate of formula **III**;

The reduction of the imine **XI** can be performed with $H_2$ in the presence of a catalyst such as Pd / C. Alternatively, a transfer hydrogenation using formates or other $H_2$-donors, which are known to the person skilled in the art, can be applied for the reduction of imine **XI:** Imine **XI** can also be reduced with complex hydrides such as $NaBH_4$ of $LiAlH_4$. In a preferred embodiment, imine **XI** is reduced with $H_2$ or a hydrogen donor such as potassium formate in the presence of Pd / C. The reduction of **XI** using Pd / C with molecular $H_2$ is described in Zhao, M. M.; McNamara, J. M.; Ho, G.-J.; Emerson, K. M.; Song, Z. J.; Tschaen, D.M.; Brands, K. M. J.; Dolling, U.-H.; Grabowski, E. J. J.; Reider, P.J., J. Org. Chem. 2002, 67, 6743-6747.

After the reduction, the catalyst is removed by filtration. In a preferred embodiment the filtrate is directly used in the next step.

Alternatively, the solvent can be removed and a solvent more suitable for the next step can be added;

> **f)** alkylation of compound **III** to give aprepitant or fosaprepitant directly or via e.g. protected intermediates;
>
> **g)** optionally, conversion of aprepitant to fosaprepitant by phosphorylation or a phosphorylation - deprotection sequence.

The alkylation is carried out using a solution of compound **III** according to prior art processes, which are described in EP 0734381 B1, WO 99065900 A1, WO 2001096315 A1, or WO 2003089429 A1.

Fosaprepitant is prepared from aprepitant or **III** as described in WO2006060110 A1 or EP 0748320 B1.

The present invention further relates to the following new compounds. These compounds are potential impurities in the process. A major advantage of the described process is that the each level of the compounds **iso-XI, iso-I,** and **iso-II** in aprepitant or fosaprepitant, respectively, is below 1%, preferably below 0.5%, most preferably below 0.1%.

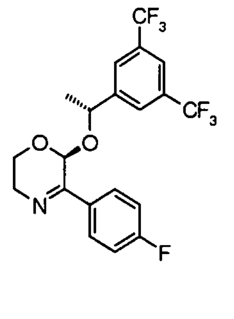

*iso*-**XI**

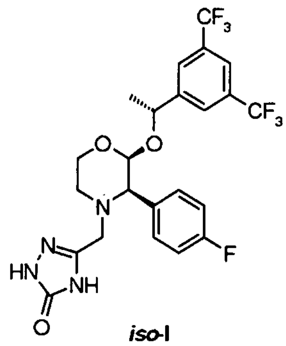

*iso*-**I**

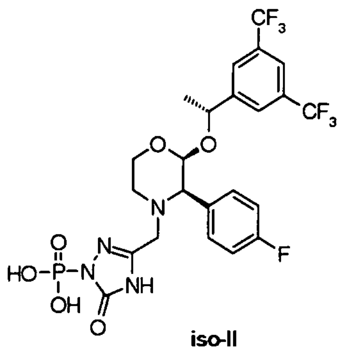

**iso-II**

These compounds may be prepared directly via the same reaction sequence as used for the synthesis of aprepitant or fosaprepitant (steps a - g), if the compound of formula iso-**VII** is isolated as an addition salt with a chiral acid in step a and the compounds of formula iso-I or iso-**II** are obtained following the subsequent reaction steps via intermediate compound of formula iso-**XI.**

EXAMPLES

The following examples describe the present invention in detail, but they are not to be construed to be in anyway limiting for the present invention.

*Example 1: Synthesis of **VII**.L-DTTA*

In a 2L reaction vessel equipped with a half-moon propeller and a thermomether 96.0mL of a 40% aqueous glyoxal solution (1.2 equivalents, 840mmol) were dissolved in 1400mL of toluene. Then 100.8mL of *N*-benzylaminoethanol (1 equivalent, 700mmol) were added. During addition the temperature rises from 25°C to 35°C. The resulting grey suspension was stirred at 35°C for 30 minutes. In the meantime 102.8g of *p*-fluor-phenyl-boronic acid (1.05 equivalents, 735mmol) were dissolved in 500mL of ethanol. A brown slightly turbid solution was formed. This solution was added to the reaction mixture within 45 minutes. The reaction was stirred at 35°C for 1.5h and then 1000mL of water were added (pH of the resulting solution = 5.8). Then 500mL of saturated aqueous sodium hydrogen carbonate solution were added (pH = 7.6) and the reaction mixture was stirred for 5min at 25°C. The layers were separated and the organic layer was washed consecutively with 500mL of a 4.3% aqueous sodium hydrogen carbonate solution and with 500mL of a 13.3% aqueous sodium chloride solution. The main part of toluene was removed under reduced pressure (70°C, 100mbar) to yield 234g of a yellow oil. This oil was dissolved in 1169g of isopropanol at 40°C. Then 149g of L-DTTA (0.55 equivalents, 385mmol) were added. The reaction mixture was heated to 55°C and seeded. The resulting suspension was cooled to 22°C and stirred over night. Then 1169mL of water were added dropwise and the mixture was allowed to crystallize for 1h at 22°C. Then the suspension was cooled to 0°C and stirred for 17h. The product is filtered off and washed twice with 200mL of a cold (0°C) mixture of isopropanol and water (1:1) to yield 221.1g of wet product (ee = 78%). In a 3L reaction vessel the crude material was dissolved in 1050mL of isopropanol at 45°C to give a clear solution. Then 1050mL of water were added under stirring at this temperature to give a clear yellow solution which was cooled slowly to 38°C and the product started to crystallize. The suspension was stirred for 45min at 38°C and was then cooled slowly to 0°C and stirred for 30min. The resulting crystals were collected by filtration, washed with 500mL of a mixture of isopropanol and water (1:1), and dried at 45°C and 20mbar to give 124g of the title compound as white crystals (ee =99%).

*Example 1a: Synthesis of **VII**.L-DTTA:*

In a 2L reaction vessel equipped with a half-moon propeller and a thermomether 96.0mL of a 40% aqueous glyoxal solution (1.2 equivalents, 840mmol) were dissolved in 1400mL of toluene. Then 100.8mL of N-benzylaminoethanol (1 equivalent, 700mmol) were added. During addition the temperature rises from 25°C to 29°C. The resulting grey suspension was stirred at 35°C for 30 minutes. In the meantime 102.8g of p-fluor-phenyl-boronic acid (1.05 equivalents, 735mmol) were dissolved in 500mL of ethanol. A brown slightly turbid solution was formed. This solution was added to the reaction mixture within 15 minutes. The reaction was stirred at 35°C for 1h (pH = 5.26) and then 500mL of a 4.3% aqueous sodium hydrogen carbonate solution were added (pH of the resulting solution = 7.85). The reaction mixture was stirred for 5min at 35°C. The layers were separated and the organic layer was washed consecutively with 500mL of a 4.3% aqueous sodium hydrogen carbonate solution and with 500mL of a 13.3% aqueous sodium chloride solution. The main part of toluene was removed under reduced pressure (60°C, 20mbar) to yield 201g of a yellow oil. This oil

was dissolved in 1792mL of isopropanol at 55°C. Then 149g of L-DTTA (0.55 equivalents, 385mmol) and 1407mL of water were added. The reaction mixture was heated to 58°C and the resulting clear solution was cooled to 22°C and seeded. The suspension was stirred over night at 10°C. The product was filtered off and washed three times with 200mL of a cold (0°C) mixture of isopropanol and water (1:1) to yield 132g of the desired product (ee = 99%). The mother liquor was concentrated under reduced pressure (80mbar) at 50°C. The aqueous residue was dissolved in dichloromethane and a 4.3% aqueous sodium hydrogen carbonate solution. A strong gas development was observed. The mixture was stirred for 15min. The layers were separated and the organic layer was washed with a 4.3% aqueous sodium hydrogen carbonate solution. Then the dichloromethane layer was concentrated under reduced pressure (10mbar) at 45°C to give an orange-brown oil. The oil was dissolved in isopropanol and water (1:1). The reaction mixture was warmed to 45$\pm$5°C and 0.5 equivalents of sodium hydroxide were added (pH 8$\pm$0.5 $\rightarrow$ 13$\pm$0.5). After 2 hours full racemization of the mixture was determined. The solution was neutralized with HCl. Then the reaction mixture was heated to 50°C and 1.1 equivalents of L-DTTA and water were added. A clear solution was formed which was cooled to 20°C and seeded. The reaction mixture was stirred over night at 10°C. The crystals were filtered off and washed 3 times with isopropanol/water (1:1). The product was dried under reduced pressure 20mbar, 40°C.

This racemization - crystallization procedure was repeated three times to give 367g (78%) of the title compound *(ee>99%).*

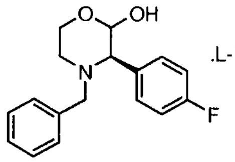
.L-DTTA

**$^1$H-NMR** (DMSO-d6, 300MHz) $\delta$ (ppm) = 2.18 (dt, $CH_2$, 1H, J 11.6Hz, J 2.9Hz), 2.40 (s, $CH_3$, 6H), 2.55 (m, $CH_2$, 1 H), 2.92 (d, $CH_2$, 1 H, J 13.4Hz), 3.06 (d, CH, 1 H, J 7.2Hz), 3.52 (d, $CH_2$, 1 H, J 13.4Hz), 3.64 (t, $CH_2$, 1 H, J 11.4Hz), 3.79 (d, CH, 1H, J 10.7Hz), 4.45 (d, CH, 1H, 7.0Hz), 5.85 (s, CH, 2H), 7.23 (m, CH, 7H), 7.40 (d, CH, 2H, J 8.0Hz), 7.51 (t, CH, 2H, J 8.0Hz), 7.92 (d, CH, 4H, J 8.0Hz).

**$^{13}$C-NMR** (DMSO-d6, 75.47MHz) $\delta$ (ppm) = 22.12, 51.27, 58.92, 64.13, 72.23, 72.28, 98.24, 115. 64, 115.92, 126.67, 127.82, 129.06, 129.35, 130.34, 130.43, 131.37, 131.47, 136.92, 138.82, 145.45, 160.71, 163.93, 165.54, 168.15.

$[\alpha]_D^{20}$ **= -77,6°** (10mg/1mL acetonitrile)

**MS:** [**VII** + H]$^+$ 288,0 (100%), [DTTA+H]$^+$ 387,3 (20%), [DTTA+$NH_4$]$^+$ 404,4 (15%), [DTTA+Na]$^+$ 409,3 (10%), [VII.DTTA+H]$^+$ 674,3 (15%).

**Mp:** 150-152°C.

*Example 2:* Compound **VII**

20g (29.7mmol) of **VII.**L-DTTA were dissolved in 200mL of toluene before 200mL of a 4.3% aqueous sodium hydrogen carbonate solution were added. The mixture was stirred at ambient temperature for 10min. The layers were separated and the organic layer was dried with sodium sulfate. After filtration the organic layer was concentrated to give 8.4g of the title compound which was used in the next step without further purification.

An analytical sample was purified by column chromatography on silica using pentane / ether as eluent.

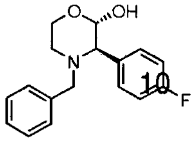

**$^1$H-NMR** ($CDCl_3$, 300MHz) $\delta$ (ppm) = 2.33 (dt, $CH_2$, 1H, J 11.6Hz, J 3.6Hz), 2.75 (td, $CH_2$, 1H, J 9.9Hz, J 2.0Hz), 2.96 (d, $CH_2$, 1H, J 13.4Hz), 3.16 (d, CH, 1H, J 7.2Hz), 3.57 (bs, OH, 1H), 3.73 (d, $CH_2$, 1H, J 13.4Hz), 3.39 (m, $CH_2$, 2H), 4.71 (d, CH, 1H, 7.1 Hz), 7.12 (t, CH, 2H, J 8.7Hz), 7.30 (m, CH, 5H), 7.54 (m, CH, 2H).

**$^{13}$C-NMR** ($CDCl_3$, 75.47MHz) $\delta$ (ppm) = 50.88, 59.15, 64.83, 72.38, 98.13, 115.76, 116.04, 127.53, 128.71, 129.13, 130.78, 130.89, 131.08, 135.00, 138.58, 161.22, 164.48.

*Example 3:* Synthesis of compound **IX**

a) Formation of the trichloroacetimidate

To a solution of **VII** free base (42.6g, 148mmol, 1 equivalent) in 150mL of toluene were added 26.6g of potassium carbonate (193mmol, 1.3 equivalents) and 25.3mL of trichloroacetonitrile (36.4g, 252mmol, 1.7 equivalents). The reaction mixture was stirred for 1 h at ambient temperature.

The suspension was filtered and the filtrate was concentrated to about 50% of the initial volume. The resulting solution was used in the next step without further purification

An analytical sample was prepared by complete evaporation of the solvent.

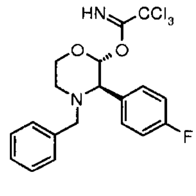

**$^1$H-NMR** ($CDCl_3$, 300MHz) $\delta$ (ppm) = 1.43 (d, $CH_3$, 3H, J 6.6Hz), 2.35 (dt, $CH_2$, 1H, J 11.7Hz, J 3.1Hz), 2.72 (d, $CH_2$, 1H, J 11.7Hz), 2.92 (d, $CH_2$, 1H, J 13.4Hz), 3.25 (d, CH, 1H, J 7.4Hz), 3.68 (d, $CH_2$, 1H, J 13.4), 3.72 (m, $CH_2$, 1H), 3.97 (dd, $CH_2$, 1H, J 11.4Hz, J 1.7Hz), 4.20 (d, CH, 1 H, J 7.4Hz), 4.98 (q, CH, 1 H, J 6.6Hz), 7.02 (t, CH, 2H, J 8.6Hz), 7.15-7.45 (m, CH aromatic, 10H), 7.71 (s, CH, 1H).

**$^{13}$C-NMR** ($CDCl_3$, 75.47MHz) $\delta$ (ppm) = 24.78, 51.12, 59.13, 64.92, 70.90, 74.25, 101.48, 115.69, 115.96, 121.98, 126.64, 127.46, 128.64, 129.05, 130.50, 131.74, 132.18, 134.52, 138.59, 145.75, 161.20, 164.46. $[\alpha]_D^{20}$ = +25.2°C (10mg/1mL acetonitril)

b) Coupling

To the concentrated mixture of trichloroacetimidate from step a) were added 40.0g (154mmol, 1 equivalent) of chiral alcohol **VIII.** After stirring for 5min at ambient temperature a clear solution was formed. The mixture was cooled to -10°C and 2.94mL (23mmol, 0.15 equivalents) of $BF_3 \cdot Et_2O$ were added dropewise at this temperature within 15min. The reaction mixture was stirred for 45min at -10°C before 600mL of a 4.3% aqueous sodium hydrogen carbonate solution was added. The layers were separated and the organic layer was washed twice with 500mL of a 4.3% aqueous sodium hydrogen carbonate solution and was then concentrated under reduced pressure (60°C, 40mbar) to yield 91.6g (98%) of the title compound which was used in the next step without further purification.

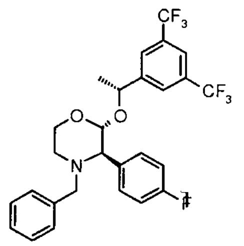

**$^1$H-NMR** ($CDCl_3$, 300MHz) $\delta$ (ppm) = 2.49 (dt, $CH_2$, 1H, J 10.6Hz, J 3.6Hz), 2.88 (td, $CH_2$, 1H, J 12.0Hz, J 2.5), 3.12 (d, $CH_2$, 1H, J 13.5Hz), 3.61 (d, CH, 1H, J 7.2Hz), 3.83 (d, $CH_2$, 1H, J 13.5Hz), 4.02 (m, $CH_2$, 1H), 4.15 (m, $CH_2$, 1H), 5.92 (d, CH, 1 H, J 7.2Hz), 7.08-7.43 (m, CHaromatic, 9H), 8.50 (s, NH, 1 H).

**$^{13}$C-NMR** ($CDCl_3$, 75.47MHz) $\delta$ (ppm) = 50.25, 58.90, 65.46, 68.94, 91.20, 99.24, 115.71, 115.99, 127.67, 128.89, 129.52, 131.20, 131.31, 133.21, 138.36, 161.36, 161.46, 164.67.

*Example 4:* Synthesis of compound **X**

108.9g (206.4mmol, 1 equivalent) of **IX** were dissolved in 360mL of methanol in a 2L round bottom flask. Then 78,5g (412.8mmol, 2 equivalents) of *para*-toluene sulfonic acid and 21.8g of Pd/C (10%) were added. The reaction

vessel was charged with hydrogen and the mixture was stirred for 16h at ambient temperature. The catalyst was filtered off and washed three times with 50mL of methanol. The filtrate was concentrated under reduced pressure (45°C, 10mbar) to yield a suspension which was dissolved in 620mL of dichloromethane and 500mL of a 4.3% aqueous sodium hydrogen carbonate solution. The layers were separated and the organic layer was washed with 250mL portions of a 4.3% aqueous sodium hydrogen carbonate solution until complete depletetion of the sulfonic acid was detected by HPLC. The organic layer was concentrated under reduced pressure (45°C, 10mbar) to give 70.8g (78%) of the title compound which was used in the next step without further purification.

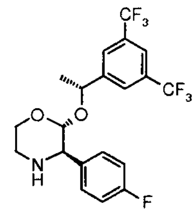

**$^1$H-NMR** (DMSO-d6, 300MHz) $\delta$ (ppm) = 1.29 (d, $CH_3$, 3H, J 6.6Hz), 2.79 (m, $CH_2$, 2H), 3.55 (d, CH, 1 H, J 7.4), 3.61 (dt, $CH_2$, 1 H, J 11.0Hz, J 2.6Hz), 3.90 (d, $CH_2$, 1 H, J 10.9 Hz), 4.07 (d, CH, 1 H, J 7.4Hz), 5.07 (q, CH, 1 H, J 6.6Hz), 7.09 (t, CH, 2H, J 8.8Hz), 7.34 (m, CH, 2H), 7.39 (s, CH, 2H), 7.76 (s, CH, 1 H).
**$^{13}$C-NMR** (DMSO-d6, 75.47MHz) $\delta$ (ppm) = 24.63, 45.34, 63.66, 66.04, 73.01, 101.84, 114.60, 114.88, 117.95, 120.94, 120.99, 121.04, 121.57, 125.18, 126.59, 128.79, 129.86, 129.96, 130.36, 130.79, 131.23, 136.48, 136.52, 146.94, 160.40, 163.63.

*Example 5:* Synthesis of **XI**

25.0g (57.1mmol, 1 equivalent) of **X** were dissolved in 39.5mL of dimethylformamide and 1.58g (11.4mmol, 0.2 equivalents) of potassium carbonate were added. Then the suspension was cooeld to - 5°C before 8.78g (65.7mmol, 1.2 equivalents) of NCS were added within 15 minutes. The reaction mixture was stirred at -5°C for 5min before 10.7mL (71.5mmol, 1.3 equivalents) of DBU were added within 20min. The reaction mixture was stirred at -5°C for 5h. Then 102mL of water and 51mL of dichloromethane were added and the layers were separated. The organic layer was washed 5 times with 100mL of a 4% aqueous lithium chloride solution and concentrated under reduced pressure (45°C, 10mbar) to yield 21.8g of an orange suspension. The suspension was dissolved at 47°C in 68.2mL of isopropanol. The mixture was cooled slowly to ambient temperature, while crystallization starts. The suspension was stirred for 45min before 17.8mL of water were added. The suspension was then cooled to 0°C and stirred for 1.5h. The crystals were collected by filtration and washed three times with 20mL of a cold mixture of isopropanol / water (3.8:1.0) to yield 16.9g (78%) of the title compound as a white crystalline product after drying at 40°C under reduced pressure.

*Example 5a:* Synthesis of **XI**

1.0g (2.29mmol, 1 equivalent) of **X** were dissolved in 1.58mL of acetonitrile and 63mg (0.46mmol, 0.2 equivalents) of potassium carbonate were added, before 8,16mL (13.7mmol, 6.0 equivalents) of NaOCl (10%) were added within 15 minutes. Then 2.22mL (14.9mmol, 6.5 equivalents) of DBU were added. The reaction mixture was stirred at ambient temperature for 14h. Then 50mL of water and 50mL of dichloromethane were added and the layers were separated and concentrated under reduced pressure (45°C, 10mbar) to yield 0.92g (92%) of **XI.**

*Example 6:* Synthesis of **XI** without isolation of intermediates

In a 2L reaction vessel equipped with a half-moon propeller and a thermomether 96.0mL of a 40% aqueous glyoxal solution (1.2 equivalents, 840mmol) were dissolved in 1400mL of toluene. Then 100.8mL of N-benzylaminoethanol (1 equivalent, 700mmol) were added. During addition the temperature rises from 25°C to 35°C. The resulting grey suspension was stirred at 35°C for 30 minutes. In the meantime 102.8g of p-fluor-phenyl-boronic acid (1.05 equivalents, 735mmol) were dissolved in 500mL of ethanol giving a brown slightly turbid solution. This solution was added to the reaction mixture within 45 minutes. The reaction stirred at 35°C for 1.5 h and then 500mL of a 8.6% aqueous sodium hydrogen carbonate solution were added to the reaction mixture (pH = 7.7) and the resulting mixture was stirred for 5min at 25°C. The layers were separated and the organic layer was washed consecutively with 500mL of a 4.3% aqueous sodium hydrogen carbonate solution and with 500mL of a 13.3% aqueous sodium chloride solution. The combined aqueous layers were reextracted with 200mL of toluene. The main part of toluene was removed under reduced pressure (70°C, 100mbar) to yield 240g of a yellow oil. The oil was dissolved in 1407g of isopropanol at 55°C. Then 148.7g of

L-DTTA (0.55 equivalents, 385mmol) were added. The reaction mixture was heated to 58°C. Then 1407mL of water were added dropwise. Then the reaction mixture was allowed to crystallize for 1 h at 58°C and then the suspension was cooled to 10°C and stirred for 12h. The product was filtered off and washed three times with 200mL of a cold (0°C) mixture of isopropanol and water (1:1) to yield 210g of wet product which was dried at 45°C and 20 mbar to give 161g of VII.L-DTTA as white crystals (ee =98%).

VII.L-DTTA was dissolved in 900mL of toluene before 2000mL of a half saturated aqueous sodium hydrogen carbonate solution were added. The mixture was stirred at ambient temperature for 10min. The layers were separated and the organic layer was concentrated to a mass of 69.5g. The resulting residue was dissolved in 487g of toluene and 43.5g of potassium carbonate (315mmol, 1.3 equivalents) and 41.2mL of trichloroacetonitrile (411 mmol, 1.7 equivalents) were added. The reaction mixture was stirred for 55min at ambient temperature. The suspension was filtered and the filtrate was concentrated to about 50% of the initial volume.

To the resulting residue 62,5g (242mmol, 1 equivalent) of VIII were added. After stirring for 5min at ambient temperature a clear solution was formed. The mixture was cooled to -10°C and 4.60mL (36mmol, 0.15 equivalents) of $BF_3 \cdot Et_2O$ were added dropewise at this temperature within 25min. The reaction mixture was stirred at -10°C for 20min before 600mL of a 4.3% aqueous sodium hydrogen carbonate solution were added. The mixture was allowed to come to ambient temperature and the layers were separated. The organic layer was washed twice with 500mL of a 4.3% aqueous sodium hydrogen carbonate solution and concentrated under reduced pressure (60°C, 40mbar) to yield 118.9g of IX which were dissolved in 396mL of methanol in a 2L round bottom flask. Then 48.5g (254.6mmol, 2 equivalents) of para-toluene sulfonic acid and 13.4g of Pd/C (10%) were added. The reaction vessel was charged with hydrogen and the reaction mixture was stirred for 5h. The catalyst was filtered off and washed three times with 50mL of methanole. The filtrate was concentrated under reduced pressure (45°C, 10mbar) to yield 153g of slightly red crystals. Then 500mL of dichloromethane were added and the organic layer was neutralized with 800mL of a 4.3% aqueous sodium hydrogen carbonate solution. The layers were separated and the organic layer was washed twice with 500mL of a 4.3% aqueous sodium hydrogen carbonate solution and was then concentrated under reduced pressure (45°C, 10mbar) to give 88g of crude **X.**

The resulting crude **X** was dissolved in 109mL of dimethylformamide and 4.36g (31.6mmol, 0.2 equivalents) of potassium carbonate were added. Then the suspension was cooeld to -11 °C before 24.3g (65.7mmol, 1.2 equivalents) of NCS were added within 15 minutes. The reaction mixture was stirred at that temperature for 5min before 29.5mL (197mmol, 1.3 equivalents) of DBU were added at -5°C in 25min. The reaction mixture was stirred for 2h at -5°C and then 280mL of water and 140mL of dichloromethane were added and the layers were separated. The organic layer was washed 5 times with 280mL of a 4% aqueous lithium chloride solution and concentrated under reduced pressure (45°C, 10 mbar) to yield in 73.9g of an orange oil. The oil was dissolved at 47°C in 96mL isopropanol. The mixture was cooled slowly to ambient temperature, while crystallization starts. The suspension was stirred for 1h at 0°C. The crystals were collected by filtration and washed three times with 50 mL of a cold mixture of isopropanol / water (3.8:1.0) to give 34.2g (50%) of the title compound after drying at 40°C under reduced pressure.

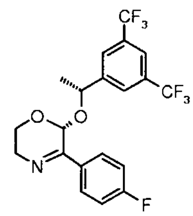

**$^1$H-NMR** ($CDCl_3$, 300MHz) $\delta$ (ppm) = 1.58 (d, $CH_3$, 3H, J 6.6Hz), 3.84 (m, $CH_2$, 1H), 3.92 (m, $CH_2$, 2H), 4.10 (m, $CH_2$, 1H), 5.13 (s, CH, 1H), 5.16 (q, CH, 1H, J 6.6Hz), 7.00 (t, CH, 2H, J 8.6Hz), 7.46 (dt, CH, 2H, J 5.5Hz, J 3.2Hz), 7.79 (s, CH, 2H), 7.90 (s, CH, 1 H).

**$^{13}$C-NMR** ($CDCl_3$, 75.47MHz) $\delta$ (ppm) = 24.35, 48.35, 56.31, 73.51, 88.78, 115.59, 115.88, 121.79, 122.48, 122.53, 122.58, 125.41, 127.61, 128.77, 128.88, 131.74, 132.19, 132.63, 133.04, 145.24, 161.98, 162.81, 166.12.

$[\alpha]_D^{20}$ = +12.7° (10mg/1mL acetonitrile)

Crystallographic data:

Data collection has been performed on an Oxford Diffraction Gemini single crystal diffractometer. A single crystal of **XI** (0.01 x 0.03 x 0.12 mm in size) was studied at 173(2) K using graphite monochromatized Cu-K□ radiation. Basic crystallographic data are as follows : monoclinic symmetry, space group $P12_11$, a = 5.5081(1)Å, b = 10.5758(2)Å,

c = 16.2303(4)Å, β = 90.977(2)°, V = 945.4(1)$Å^3$, chemical formula $C_{20}H_{16}F_7NO_2$, Z = 2. The structure was solved by direct methods and refined to a residual of R(|F|)=0.048 for 2237 independent observed reflections (I > 2σ(I)) and 273 parameters.

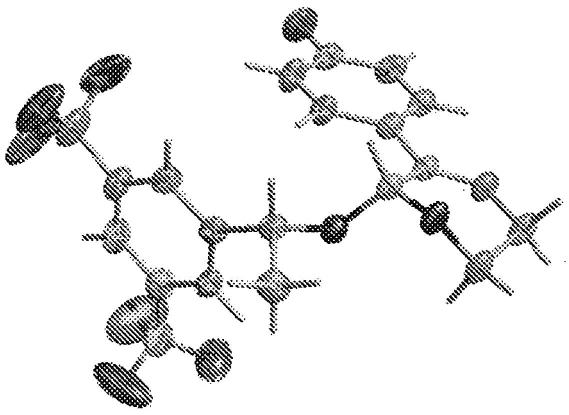

*Example* 7: Synthesis of **III**

22.2g (51 mmol, 1 equivalent) of **XI** were dissolved in 330mL of methanol. Then 2.78g of Pd/C (10%) were added. The flask was charged with hydrogen and the resulting mixture was stirred for 2h at ambient temperature. The catalyst was filtered off and washed three times with 50mL of methanol. The filtrate was concentrated under reduced pressure (45°C, 10mbar) to yield 12.4g (55.5%) of the title compound as a colourless oil which was used without further purification in the next step.

*Example 7a:* Synthesis of **III**

100mg (0.23mmol, 1 equivalent) of **XI** were dissolved in 1.3mL of ethanol. Meanwhile 56mg (0.67mmol, 2.9 equivalents) of potassium formate were dissolved in 0.09mL of water and 21 mg of Pd/C (20%) were added. Then the solution of **XI** in ethanol was added. The resulting mixture was stirred for 2h at ambient temperature. The catalyst was filtered off and washed three times with 1 mL of methanol. The filtrate was concentrated under reduced pressure (45°C, 10mbar) to yield 86mg (86%) of the title compound as a colourless oil.

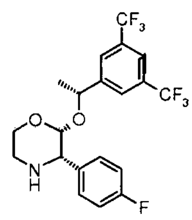

**$^1$H-NMR** (DMSO-$d_6$, 300MHz) δ (ppm) = 1.36 (d, $CH_3$, 3H, J 6.6Hz), 2.97 (m, $CH_2$, 2H), 3.50 (d, CH, 1 H, J 7.42), 3.92 (d, CH, 1 H, J 2.3Hz), 3.99 (m, $CH_2$, 1 H), 4.42 (d, CH, 1 H, J 2.3Hz), 4.97 (q, CH, 1 H, J 6.6Hz), 7.03 (t, CH, 2H, J 8.4Hz), 7.35 (dt, CH, 2H, J 5.8Hz, J 2.8Hz), 7.39 (s, CH, 2H), 7.80 (s, CH, 1 H).
**$^{13}$C-NMR** (DMSO-$d_6$, 75.47MHz) δ (ppm) = 24.68, 45.76, 59.21, 61.36, 71.82, 95.69, 114.52, 114.80, 121.13, 122.61, 125.23, 126.71, 128.84, 129.11, 129.21, 129.86, 130.29, 130.73, 131.16, 136.39, 136.43, 147.27, 160.06, 163.28.

*Example 8:* Synthesis of **I**

5-Chloromethyl-2,4-dihydro-[1,2,4]triazol-3-one-variant:

To a solution of 0.60g (1.48mmol, 1 equivalent) of **III** in 3.1mL of DMF were added 226mg (1.64mmol, 1.1 equivalents) of potassium carbonate at ambient temperature. The mixture was stirred at 20°C and a solution of 238mg (1.78mmol, 1.2 equivalents) of 5-Chloromethyl-2,4-dihydro-[1,2,4]triazol-3-one in 1.5mL of DMF was added dropwise within 15min. The reaction was stirred for 15min at 20°C before 10mL of water were added dropwise while the product started to crystallize. The resulting suspension was stirred for 10min at 25°C before it is cooled to 0°C and stirred for

1h. The crystals was collected by filtration and washed with cold water to give 416mg (68%) of the title compound after drying under reduced pressure (40°C, 10mbar) as a white crystalline product.

*Example 8a:* Synthesis of I

*N*'-[1-Amino-2-chloro-eth-(Z)-ylidene]-hydrazinecarboxylic acid methyl ester-variant:

To a solution of 500mg (1.14mmol, 1 equivalent) of **III** in 4.2mL of acetonitrile were added 546μL (3.29mmol, 2.9 equivalents) of *N,N*-diisopropylethylamine and 244mg (1.48mmol, 1.29 equivalents) of *N*-[1-Amino-2-chloro-eth-(Z)-ylidene]-hydrazinecarboxylic acid methyl ester. The resulting suspension was stirred at ambient temperature for 3h while a clear solution was formed. The reaction mixture was concentrated under reduced pressure (45°C, 100mbar) and the residue was dissolved in 10mL of dichloromethane and washed with 10mL of a 26.5% aqueous sodium chloride solution. The organic layer was concentrated under reduced pressure (45°C, 100mbar). Then 4.2mL of acetonitrile were added to the residue and the mixture was transferred to a reactor where it was stirred for 55h at 110°C and 1.5bar. Then the reaction mixture was concentrated under reduced pressure (45°C, 10 mbar) and the residue was dissolved in 5.6mL of methanol. The reaction mixture was heated to reflux and charcoal was added.

The reaction mixture was kept at reflux for 30min before it was filtered over a bed of celite and washed with methanol. The filtrate was concentrated under reduced pressure and then suspended in acetonitrile. The resulting crystalline product **I** was collected by filtration and washed with cold acetonitrile to give 324mg (53%) of the title compound as a white crystalline product.

Example 9: Synthesis of I without isolation of intermediates:

A mixture of 33.5g (77mmol, 1 equivalent) of **XI** dissolved in 496mL of methanol and 6.69g of Pd/C (10%) was charged with hydrogen and stirred for 2h at ambient temperature. The catalyst was filtered off and washed three times with 50mL of methanol. The filtrate was concentrated under reduced pressure (45°C, 10mbar) to yield in 34g of **III** as a colourless oil. This oil was dissolved in 278mL of acetonitrile and 28g (218mmol, 2.9 equivalents) of *N,N*-diisopropylethylamine and 16g (97,8mmol, 1.3 equivalents) of *N*'-[1-Amino-2-chloro-eth-(Z)-ylidene]-hydrazinecarboxylic acid methyl ester were added. The mixture was stirred at ambient temperature for three hours and then concentrated under reduced pressure. The residue was dissolved in 300mL of dichloromethane and washed with 300mL of a 26.5% aqueous sodium chloride solution. The organic layer was concentrated under reduced pressure (45°C, 10mbar). Then 130mL acetonitrile were added and the resulting mixture was transferred to a reactor where it was stirred for 45h at 110°C and 1.5 bar. The reaction mixture was then concentrated under reduced pressure (45°C, 10mbar) and the residue was dissolved in 371mL methanol. The reaction mixture was heated to reflux and charcoal was added. The reaction mixture was kept at reflux for 30min before it was filtered over a bed of celite and washed with methanol. The filtrate was concentrated under reduced pressure and then suspended in 278mL of acetonitrile. The resulting crystals were collected by filtration and washed with acetonitrile to give 377g (69%) of the title compound as a white crystalline product.

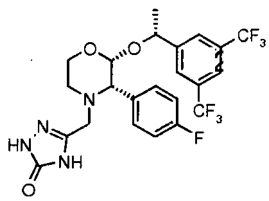

NMR: **$^1$H-NMR** (DMSO-$d_6$, 300MHz) δ (ppm) = 1.36 (d, $CH_3$, 3H, J 6.5Hz), 2.39 (dt, $CH_2$, 1H, J 11.7Hz, J 3.1 Hz), 2.75 (d, $CH_2$, 1H, J 14.2Hz), 2.84 (d, $CH_2$, 1H, J 11.7Hz), 3.38 (d, $CH_2$, 1H, J 13.9Hz), 3.49 (d, CH, 1H, J 2.54), 3.62 (d, $CH_2$, 1H, J 10.9Hz), 4.12 (t, $CH_2$, 1H, J 9.9Hz), 4.33 (d, CH, 1H, J 2.7Hz), 4.94 (q, CH, 1H, J 6.5Hz), 7.07 (t, CH, 2H, J 8.8Hz), 7.37 (s, CH, 2H), 7.51 (t, CH, 2H, J 6.1 Hz), 7.83 (s, CH, 1 H), 11.29 (bs, NH, 2H).

**$^{13}$C-NMR** (DMSO-$d_6$, 75.47MHz) δ (ppm) = 24.75, 50.79, 51.88, 59.07, 68.02, 71.87, 95.77, 114.79, 115.07, 121.39, 121.60, 125.22, 126.87, 128.83, 129.88, 130.31, 130.74, 131.18, 131.37, 131.47, 133.52, 133.56, 144.24, 146.87, 156.72, 160.45, 163.68.

*Example10:* Synthesis of ***iso*-VII.D-DTTA**

In a 2L reaction vessel equipped with a half-moon propeller and a thermomether 96.0mL of a 40% aqueous glyoxal solution (1.2 equivalents, 840mmol) were disolved in 1400mL of toluene. Then 100.8mL of *N*-benzylaminoethanol

(1 equivalent, 700mmol) were added. During addition the temperature rises from 25°C to 35°C. The resulting grey suspension was stirred at 35°C for 30 minutes. In the meantime 102.8g of *p*-fluor-phenyl-boronic acid (1.05 equivalents, 735mmol) were dissolved in 500mL of ethanol. A brown slightly turbid solution was formed. This solution was added to the reaction mixture within 45 minutes. The reaction was stirred at 35°C for 1.5h and then 1000mL of water were added (pH of the resulting solution = 5.8). Then 500mL of saturated aqueous sodium hydrogen carbonate solution were added (pH = 7.6) and the reaction mixture was stirred for 5min at 25°C. The layers were separated and the organic layer was washed consecutively with 500mL of a 4.3% aqueous sodium hydrogen carbonate solution and with 500mL of a 13.3% aqueous sodium chloride solution. The main part of toluene was removed under reduced pressure (70°C, 100mbar) to yield 234g of a yellow oil. This oil was dissolved in 1169g of isopropanol at 40°C. Then 149g of D-DTTA (0.55 equivalents, 385mmol) were added. The reaction mixture was heated to 45°C and 1620mL of water were added dropwise at that temperature. The resulting mixture was cooled to 10°C while crystallization started. The resulting suspension was stirred for 1h at 10°C and the crystals were filtered off and washed three times with 330mL of a cold (0°C) mixture of isopropanol and water (1:1) to yield 115g of wet product (ee = 87%). In a 2L reaction vessel the crude material was dissolved in 575mL of isopropanol and 6.3g (0.1 equivalents) of D-DTTA were added and the mixture was heated to 50°C. Then 575mL of water were added under stirring at 45°C to give a clear yellow solution which was slowly cooled to 20°C while crystallization starts. The resulting suspension was stirred for 45min at 20°C and was then cooled slowly to 10°C and stirred for 1h. The resulting crystals were collected by filtration, washed with 500mL of a mixture of isopropanol and water (1:1), and dried at 45°C and 20mbar to give 72.8g (68.6%) of the title compound as white crystals (ee =99.9%).

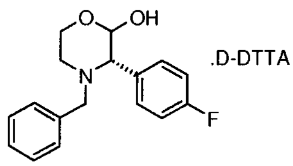

**$^1$H-NMR** ($CDCl_3$, 300MHz) δ (ppm) = 2.18 (dt, $CH_2$, 1H, J 11.4Hz, J 2.6Hz), 2.38 (s, $CH_3$, 6H), 2.58 (d, $CH_2$, 1H, J 11.7Hz), 2.92 (d, $CH_2$, 1H, J 13.4Hz), 3.07 (d, CH, 1 H, J 7.2Hz), 3.52 (d, $CH_2$, 1 H, J 13.4Hz), 3.64 (t, $CH_2$, 1 H, J 11.5Hz), 3.79 (d, CH, 1H, J 10.2Hz), 4.46 (d, CH, 1H, 7.2Hz), 5.85 (s, CH, 2H), 7.23 (m, CH, 7H), 7.39 (d, CH, 2H, J 8.0Hz), 7.51 (dt, CH, 2H, J 6.0Hz, J 2.0), 7.93 (d, CH, 4H, J 8.0Hz).

**$^{13}$C-NMR** ($CDCl_3$, 75.47MHz) δ (ppm) = 21.60, 50.77, 58.43, 63.62, 71.78, 97.73, 115. 14, 115.42, 126.19, 127.33, 127.59, 128.56, 128.86, 129.34, 129.84, 129.91, 130.88, 130.98, 131.68, 131.79, 136.34, 136.38, 138.27, 144.93, 160.22, 163.44, 165.07, 167.68.

*Example 11: **iso**-***IX**

***Iso*-VII** free base was prepared from ***iso*-VILD-DTTA.** For this purpose 68g (100.9mmol) of **D**-DTTA are dissolved in 150mL of toluene before 450mL of a 4.3% aqueous sodium hydrogen carbonate solution are added (pH = 7.1). The mixture is stirred at ambient temperature for 15 min, while $CO_2$ is formed. The layers are separated and the organic layer is washed two times with 150mL of 2.9% aqueous sodium hydrogen carbonate solution and once with 100 mL of 13.3% saturated aqueous sodium chloride solution. The organic layer is concentrated to 28.6g.

It is also possible to isolate ***iso*-VII** free base by removing toluene under reduced pressure. Then the product can be crystallized by adding 550 mL of n-heptane. Then the mixture is warmed up to 93°C and a clear solution is formed. Then the reaction is allowed to cool down again, the crystallization starts at 71 °C. The suspension is stirred for 60min at 35°C and then for 30min at 10°C. The product is filtered off and washed two times with 150mL of cold n-heptane. The product is dried under reduced pressure at 45°C to yield in 22.8g (78.6%, de: >99.9%) of the white product ***iso*-VII.**

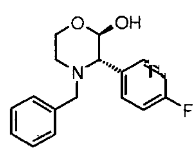

**$^1$H-NMR** ($CDCl_3$, 300MHz) δ (ppm) = 2.33 (dt, $CH_2$, 1H, J 11.6Hz, J 3.6Hz), 2.75 (d, $CH_2$, 1H, J 11.3Hz), 2.97 (d, $CH_2$, 1H, J 13.4Hz), 3.16 (d, CH, 1H, J 7.2Hz), 3.74 (d, $CH_2$, 1H, J 13.4Hz), 3.89 (m, $CH_2$, 2H), 4.70 (d, CH, 1H, 6.8Hz), 7.13 (t, CH, 2H, J 8.7Hz), 7.32 (m, CH, 5H), 7.54 (m, CH, 2H).

**$^{13}$C-NMR** ($CDCl_3$, 75.47MHz) δ (ppm) = 50.92, 59.27, 64.87, 72.33, 98.14, 115.75, 116.03, 127.56, 128.73, 129.18,

130.82, 130.89, 131.06, 135.10, 138.61, 161.21, 164.47.

*Example 12: activated **iso**-***VII***

To ***iso*-VII** free base (10.0g 36mmol 1 equivalent in 45mL toluene) 6.45g of potassium carbonate (47mmol, 1.3 equivalents) and 6.12mL of trichloroacetonitrile (61mmol, 1.7 equivalents) are added at 35°C. The reaction mixture is stirred for 3.5h at 35°C.

Then potassium carbonate is filtered off and washed two times with 10mL toluene. The mixture is concentrated to about 50% of the initial volume. Optionally, toluene is removed completely. The residue corresponds to the structure of activated ***iso*-VII.**

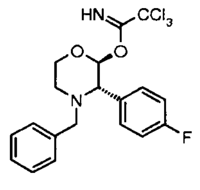

**$^1$H-NMR** ($CDCl_3$, 300MHz) $\delta$ (ppm) = 2.32 (dt, $CH_2$, 1H, J 10.6Hz, J 3.5Hz), 2.71 (td, $CH_2$, 1H, J 12.0Hz, J 2.4), 2.95 (d, $CH_2$, 1H, J 13.5Hz), 3.44 (d, CH, 1H, J 7.2Hz), 3.66 (d, $CH_2$, 1H, J 13.5Hz), 3.86 (dt, $CH_2$, 1H, J 10.7Hz, J 2.5Hz), 3.98 (m, $CH_2$, 1H), 5.54 (d, CH, 1H, J 7.2Hz), 6.94 (t, CH, 2H, J 8.7Hz), 7.18 (m, CH, 5H), 7.44(m, CH, 2H), 8.32 (s, NH, 1 H).
**$^{13}$C-NMR** ($CDCl_3$, 75.47MHz) $\delta$ (ppm) = 50.22, 58.86, 65.33, 68.91, 91.20, 99.21, 115.68, 115.91, 127.50, 128.79, 129.12, 131.17, 131.28, 133.18, 138.33, 161.34, 161.37, 164.63.

*Example 13: **iso**-***IX***

To the concentrated mixture of trichloroacetimidate from example 12 tetrahydrofuran (10ml) and 9.27g ( 36mmol, 1 equivalent) of **VIII** are added. After stirring for 5min. at ambient temperature a clear solution is formed. The mixture is cooled down to -5°C, and 1.36mL (11 mmol, 0.3 equivalents) $BF_3$*$Et_2O$ are added dropwise at this temperature within 15min. The reaction mixture is stirred at this temperature for 45min., before a 4.3% aqueous sodium hydrogen carbonate solution is added. The layers are separated and the organic layer is washed with 100mL of an 26.5% aqueous sodium chloride solution. The product layer is concentrated under reduced pressure (60°C, 50mbar) to yield in 17.22g (91%) of ***iso*-IX,** which is transferred without any purification to ***iso*-X.**

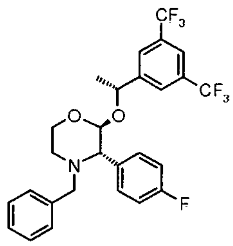

**$^1$H-NMR** ($CDCl_3$, 300MHz) $\delta$ (ppm) = 0.89 (d, $CH_3$, 3H, J 6.5Hz), 2.23 (dt, $CH_2$, 1 H, J 11.8Hz, J 3.4Hz), 2.64 (d, $CH_2$, 1H, J 11.8Hz), 2.88 (d, $CH_2$, 1H, J 13.4Hz), 3.17 (d, CH, 1H, J 7.4Hz), 3.64 (dt, $CH_2$, 1H, J 8.6Hz, J 2.3Hz), 3.68 (d, $CH_2$, 1H, J 13.4Hz), 3.79 (m, CH2, 1 H), 4.42 (d, CH, 1 H, J 7.3Hz), 4.59 (q, CH, 1 H, J 6.4Hz), 7.04 (t, CH, 2H, J 8.7Hz), 7.19 (m, CH, 5H), 7.47 (m, CH, 2H), 7.60 (s, CH, 2H), 7.66 (s, CH, 1 H).
**$^{13}$C-NMR** ($CDCl_3$, 75.47MHz) $\delta$ (ppm) = 24.78, 51.12, 59.13, 64.92, 70.90, 74.25, 101.48, 115.69, 115.96, 121.98, 126.64, 127.46, 128.64, 129.05, 130.50, 131.74, 132.18, 134.52, 138.59, 145.75, 161.20, 164.46. $[\alpha]_D^{20}$ = +25.2° (10mg/1mL acetonitrile)

*Example 14:* Synthesis of ***iso*-X**

17.22g (32.6mmol, 1 equivalent) of ***iso*-IX** are dissolved in 70mL of methanol in a 250mL round bottom flask.

Then 9.93g (52mmol, 1.6 equivalents) of para-toluene sulfonic acid and 2.8g (Pd/C 10%) are added. The reaction mixture is hydrogenated in 2.5h using a hydrogen ballon. The catalyst is filtered of and washed three times with 10mL of methanol. The filtrate is concentrated under reduced pressure (50°C, 100mbar) to yield an oil. Then 100mL of dichloromethane are added and the organic layer is neutralized with 100mL of a 4.3% aqueous sodium hydrogen carbonate solution. The layers were separated and the organic layer is washed once with 100mL of a 4.3% aqueous sodium hydrogen carbonate solution and once with 50 mL of a 26.5% aqueous sodium chloride solution, before the product is concentrated under reduced pressure (45°C, 10 mbar). 15.13 g (>99%) of the crude product ***iso*-X** were isolated.

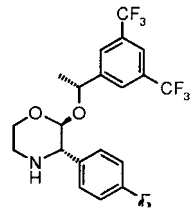

**$^1$H-NMR** (DMSO-$d_6$, 300MHz) $\delta$ (ppm) = 0.99 (d, $CH_3$, 3H, J 6.4Hz), 2.78 (m, $CH_2$, 2H), 3.54 (d, CH, 1 H, J 7.3), 3.73 (m, $CH_2$, 2H), 4.46 (d, CH, 1 H, J 7.4Hz), 4.90 (m, CH, 1 H), 7.13 (t, CH, 2H, J 8.9Hz), 7.48 (dt, CH, 2H, J 5.8Hz, J 2.5Hz), 7. 90 (s, CH, 2H), 8.01 (s, CH, 1 H).
**$^{13}$C-NMR** (DMSO-$d_6$, 75.47MHz) $\delta$ (ppm) = 21.96, 45.18, 63.61, 66.04, 67.34, 74.17, 102.50, 114.58, 115.35, 120.90, 120.95, 121.86, 125.47, 126.39, 126.80, 130.13, 130.18, 130.37, 130.56, 130.61, 136.95, 136.99, 147.90, 151.28, 160.23, 163.45.

*Example 15:* Synthesis ***iso*-XI**

9.39g (21.5mmol, 1 equivalent) of ***iso*-X** are dissolved in 40mL of dimethylformamide and 0.59g (4. 3mmol, 0.2 equivalents) of potassium carbonate are added. Then the suspension is cooled down to10°C, before 3.30g (24.7mmol, 1.2 equivalents) of NCS are added in 15 minutes. The reaction mixture is stirred at that temperature for 5min more, before 10.7mL (26.5mmol, 1.3 equivalents) of DBU are added at -10°C in 20 min. The reaction mixture is stirred for 30min at this temperature. Then 102mL of water and 51 mL of dichloromethane were added and the layers were separated. The organic layer is washed 5 times with 100mL of a 4% aqueous lithium chloride solution. The organic layer is concentrated under reduced pressure (45°C, 10mbar) to yield 9.4g of an orange oil. The product is dissolved at 47°C in 26.3mL isopropanol. The mixture is cooled down slowly to ambient temperature, while it starts crystallizing. The suspension is stirred for 45min before 8.05mL of water were added. The suspension is stirred for 1h at 0°C. The product is filtered of and washed two times with 20mL of a cold mixture of iso-propanol/water (3.8/1.0). The white crystalline product is dried at 40°C under reduced pressure, to yield in 9.87g (49%) of the product ***iso*-XI.**

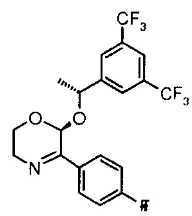

**$^1$H-NMR** (DMSO-$d_6$, 300MHz) $\delta$(ppm) = 1.50 (d, $CH_3$, 3H, J 6.5Hz), 3.36 (m, $CH_2$, 1H), 3.50 (m, $CH_2$, 1H), 3.70 (d, CH, 1H, J 3.8Hz), 5.24 (q, CH, 1H, J 6.5Hz), 5.80 (s, CH, 1 H), 7.27 (t, CH, 2H, J 8.9Hz), 7.92 (dt, CH, 2H, J 8.9Hz, J 3.3Hz), 7.97 (s, CH, 1 H), 8.03 (s, CH, 2H).
**$^{13}$C-NMR** (DMSO-$d_6$, 75.47MHz) $\delta$ (ppm) = 22.82, 47.66, 55.39, 75.25, 90.07,115.33, 115.62, 118.26, 121.15, 121.20, 121.25, 121.88, 125.49, 127.07, 127.47, 129.11, 129.19, 129.30, 129.84, 130.27, 130.70, 131.14, 132.95, 132.99, 148.05, 160.98, 162.01, 165.30.

*Example 16:* Synthesis of ***iso*-III**

4.42g (10mmol, 1 equivalent) of ***iso*-XI** are dissolved in 100mL of methanol. Then 0.88g of (Pd/C 10%) are added. The reaction mixture is hydrogenated in 16h using a hydrogen ballon. The catalyst is filtered of and washed three times with 25mL of methanol. The filtrate is concentrated under reduced pressure (45°C, 10mbar) to yield 4.38g (98.7%) of the product ***iso*-III** as a colourless oil. The crude product is used without any purification for the next reaction step.

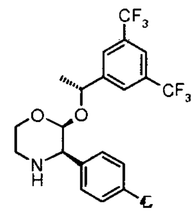

**$^1$H-NMR** (DMSO-$d_6$, 300MHz) $\delta$ (ppm) = 1.02 (d, $CH_3$, 3H, J 6.4Hz), 2.88 (m, $CH_2$, 2H), 3.30 (dd, $CH_2$, 1 H, J 10.6Hz, J 1.9Hz), 3.63 (dt, $CH_2$, 1 H, J 11.0Hz, J 3.6Hz), 4.00 (d, CH, 1 H, J 2.3 Hz), 4.78 (q, CH, 1 H, J 6.4Hz), 4.91 (d, CH, 1H, J 2.6Hz), 7.12 (t, CH, 2H, J 8.9Hz), 7.49 (m, CH, 2H), 7.92 (s, CH, 1H), 7.95 (s, CH, 2H).
**$^{13}$C-NMR** (DMSO-$d_6$, 300MHz) $\delta$ (ppm) = 21.97, 45.73, 59.39, 61.41, 73.22, 96.08, 114.42, 114.70, 118.28, 120.90, 120.95, 121.00, 121.89, 125.51, 126.88, 126.91, 129.12, 129.49, 129.60, 129.77, 130.20, 130.63, 131.07, 136.89, 136.93, 148.24, 159.90, 163.11.

*Example 17*: Synthesis of ***iso*-I**

*N'*-[1-Amino-2-chloro-eth-(Z)-ylidene]-hydrazinecarboxylic acid methyl ester-variant:

3.80g (8.71mmol, 1 equivalent) iso-**III** are dissolved in 32mL acetonitrile, before 4.29mL (25.07mmol, 2.9 equivalents) *N,N*-Diisopropylethylamine and 1.86g (11.23mmol, 1.29 equivalents) *N'*-[1-Amino-2-chloro-eth-(Z)-ylidene]-hydrazinecarboxylic acid methyl ester are added. The suspension is stirred for 3h, while a clear solution is formed. The reaction mixture was concentrated under reduced pressure (45°C, 100mbar) and the residue was dissolved in 100mL dichloromethane and washed with 100 mL of a 26.5% aqueous sodium chloride solution. The organic layer is concentrated under reduced pressure. Then 34mL acetonitrile are added. The mixture is transferred to a reactor where it is stirred for 24h at 95°C and 1.5bar. The reaction mixture is concentrated under reduced pressure (45°C, 10mbar) and the residue is dissolved in 42mL methanol. The reaction mixture is brought to reflux and a spoonful of charcoal is added. The reaction mixture is stirred at this temperature for 30min. The product is filtered and the filter is washed with methanol. The product is concentrated under reduced pressure. The reaction yields 4.94g (100%) of a slightly yellow crystalline product ***iso*-I.**

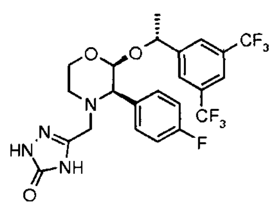

NMR: **$^1$H-NMR** (DMSO-$d_6$, 300MHz) $\delta$ (ppm) = 0.98 (d, $CH_3$, 3H, J 6.3Hz), 2.33 (dt, $CH_2$, 1 H, J 11.4Hz, J 2.7Hz), 2.73 (d, $CH_2$, 1H, J 11.6Hz), 2.84 (d, $CH_2$, 1 H, J 13.9Hz), 3.40 (m, $CH_2$, 3Hz), 3.85 (t, $CH_2$, 1H, J 6.9Hz), 4.65 (q, CH, 1H, J 6.1 Hz), 4.85 (d, CH, 1H, J 2.6Hz), 7.14 (t, CH, 2H, J 8.8Hz), 7.60 (dt, CH, 2H, J 6.0Hz, J 2.1 Hz), 7.91 (s, CH, 2H), 7.95 (s, CH, 1 H), 11.29 (s, NH, 1 H), 11.41 (bs, NH, 1 H).
**$^{13}$C-NMR** (DMSO-$d_6$, 75.47MHz) $\delta$ (ppm) = 22.28, 51.17, 51.57, 60.12, 68.32, 74.25, 97.33, 115.12, 115.40, 121.67, 122.39, 126.00, 127.49, 129.61, 130.27, 130.71, 131.14, 131.57, 131.87, 132.27, 132.38, 134.34, 144.63, 148.51, 157.12, 160.79, 164.01.

## Claims

**1.** A morpholine derivative of formula VII or formula iso-VII as an addition salt with a chiral acid wherein R1 is benzyl, substituted benzyl or another nitrogen protecting group.

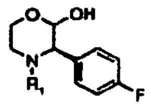

.chiral acid

VII

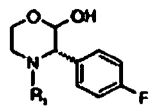

.chiral acid

iso-VII

**2.** The compounds of claim 1 , wherein the chiral acid is selected from the group consisting of tartaric acid or tartaric acid derivatives, camphersulfonic acid derivatives such as 3-bromocamphor-10-sulfonic acid, camphanic acid, 10-camphorsulfonic, or camphoric acid, amino acids such as glutamic acid, valine, or aspartic acid, mandelic acid or mandelic acid derivatives such as $\alpha$-methoxy-$\alpha$-trifluoromethylphenylacetic or $\alpha$-methoxyphenylacetic acid, acetoxy-5-etienic acid, malic acid, menthyloxyacetic acid, *N*-(a-methylbenzyl)succinamidic acid, *N*-[1-(1-naphthyl)ethyl]succinamic acid, *N*-(1-phenylethyl)succinamic acid, 1-mono-menthyl phthalate, *N*,*N*-Bis[1-phenylethyl]phthalamic acid, *N*-(1-phenylethyl)phthalamic acid, 2-phenylpropionic acid, phenylcarbamoyloxypropionic acid, pyroglutamic acid, quinic acid, 1,4-benzodioxane-2-carboxylic acid, 1,1'-binaphthalene-2,2'-diyl hydrogen phosphate, or 5-oxo-2-tetrahydrofurancarboxylic acid in either enantiomeric or diastereomeric form.

**3.** The compounds of claims 1 - 2, wherein the chiral acid is tartaric acid or a tartaric acid derivative such as di-O,O'-toluoyl tartaric acid, di-O,O'-benzoyl tartaric acid, di-O,O'-anisoyl tartaric acid, or O,O'-dibenzoyl tartaric acid mono(dimethylamide).

**4.** The compounds of claims 1 - 3, wherein the tartaric acid derivative is di- O,O'-toluoyl tartaric acid.

**5.** The compound of formula **VII** according to claim 1, wherein the chiral acid is *L*-di-O,O'-toluoyl, tartaric acid and $R_1$ is benzyl.

**6.** The compound of formula **iso-VII** according to claim 1 , wherein the chiral acid is *D*-di- O,O'-toluoyl tartaric acid and $R_1$ is benzyl.

**7.** A process for the preparation of morpholine derivatives according to claim 1 comprising the steps of

- coupling an amino alcohol of formula VI, 4-fluorophenylboronic acid or a $C_{1-6}$ alkyl or cyclic ester thereof, and glyoxal
- crystallization of the obtained morpholine derivative acid as an addition salt with a chiral acid
- isolation of a product of formula **VII**.chiral acid or of formula iso-**VII**.chiral acid
- optionally razemizing the undesired isomer and resubjecting the razemized morpholine derivative to a crystallization with a chiral acid.

**8.** The process of claim 7, wherein the chiral acid is selected from the group consisting of tartaric acid or tartaric acid derivatives, camphorsulfonic acid derivatives such as 3-bromocamphor-10-sulfonic acid, camphanic acid, 10-camphorsulfonic, or camphoric acid, amino acids such as glutamic acid, valine, or aspartic acid, mandelic acid or mandelic acid derivatives such as a-methoxya-trifluoromethylphenylacetic or a-methoxyphenylacetic acid, acetoxy-5-etienic acid, malic acid, menthyloxyacetic acid, *N*-(a-methylbenzyljsuccinamidic acid, *N*-[1-(1-naphthyl)ethyl]succinamic acid, *N*-(1-phenylethyl)succinamic acid, 1-mono-menthyl phthalate, *N*,*N*-Bis[1-phenylethyl]phthalamic acid, N-(1-phenylethyl)phthalamic acid, 2-phenylpropionic acid, phenylcarbamoyloxypropionic acid, pyroglutamic acid, quinic acid, 1,4- benzodioxane-2-carboxylic acid, 1,1 '-binaphthalene-2,2'-diyl hydrogen phosphate, or 5-oxo-2-tetrahydrofurancarboxylic acid in either enantiomeric or diastereomeric form.

**9.** The process of claim 7 - 8, wherein the chiral acid tartaric acid or a tartaric acid derivative such as di-O,O'-toluoyl tartaric acid, di-O,O'-benzoyl tartaric acid, di-O,O'-anisoyl tartaric acid, or O,O'-dibenzoyl tartaric acid mono(dimethylamide).

**10.** The process of claims 7 - 9, wherein the tartaric acid derivative is di-O,O'-toluoyl tartaric acid.

**11.** The process of claim 7, wherein the isolated product is of formula **VII**.*L*-di-O,O'-toluoyl tartaric acid or formula iso-**VII**.*D*-di-O,O'-toluoyl tartaric acid.

**12.** The process of claim 11 , wherein $R_1$ is benzyl or substituted benzyl.

**13.** Use of the compound of formula **VII**.*L*-di-O,O'-toluoyl tartaric acid in the synthesis of aprepitant or fosaprepitant.

**14.** Use of the compound of formula iso-VII.*D*-di-0,0'-toluoyl tartaric acid in the synthesis of the compounds of formula **iso-I** or **iso-II.**

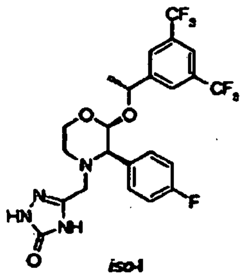
iso-I

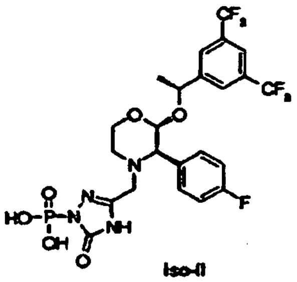
iso-II

**15.** A process for the preparation of aprepitant (**I**) or fosaprepitant (**II**), comprising the steps of

a) a three component coupling of an amino alcohol of formula **VI,** of 4-fluorophenylboronic acid or a $C_{1-6}$ alkyl or cyclic ester thereof (formula **V**), and of glyoxal (**IV**) followed by crystallization of the obtained morpholine derivative as addition salt with a chiral acid and isolation of a product of formula **VII**.chiral acid;
b) generation of hemi-acetal **VII** free base by portioning compound of formula **VII**.chiral acid between an alkaline aqueous layer and a water-immiscible organic phase; activation of the hemi-acetal functionality by transforming the OH-group into an activated derivative; reaction of the resulting activated acetal with alcohol of formula **VIII.** Removal of impurities by extraction to get a solution of compound **IX;**
c) removal of the *N*-protecting group to give the amine of formula **X;**
d) oxidation of the amine of formula **X** to the corresponding cyclic imine of formula **X** and isolation of the cyclic imine of formula **XI;**
e) reduction of imine **XI** with a catalyst and $H_2$ or an $H_2$ equivalent; removal of the catalyst by filtration to get the key intermediate of formula **III;**
f) alkylation of compound of formula **III** to give aprepitant or fosaprepitant directly or *via* e.g. protected intermediates;
g) optionally, conversion of aprepitant to fosaprepitant by phosphorylation or a phosphorylation - deprotection sequence.

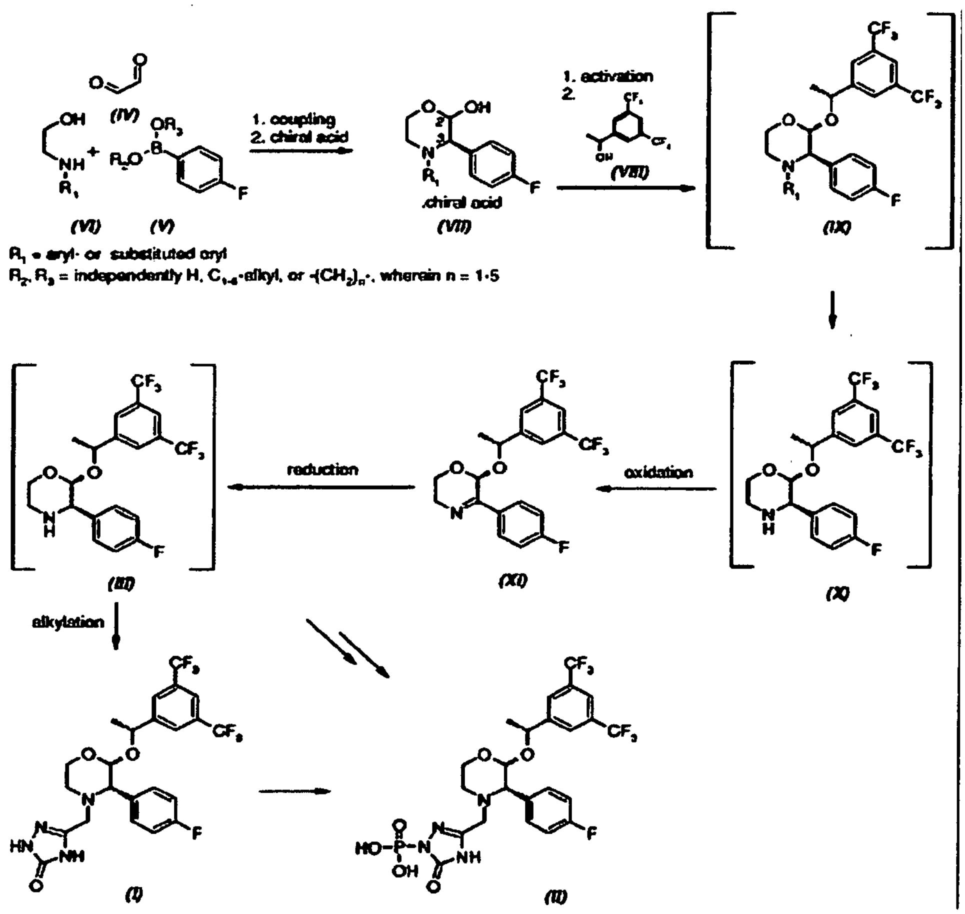


**16.** The process according to claim 15, **characterized in that** the isolated intermediates are the compounds of formula **VII**.chiral acid and of formula **XI**.

**17.** A process for the preparation of a compound of formula iso-**I** or iso-**II**, comprising the steps of

a) a three component coupling of an amino alcohol of formula **VI,** of 4-fluorophenylboronic acid or a $C_{1-6}$ alkyl or cyclic ester thereof (formula **V**), and of glyoxal (**IV**) followed by crystallization of the obtained morpholine derivative as addition salt with a chiral acid and isolation of a product of formula iso-**VII**.chiral acid;
b) generation of hemi-acetal iso-**VII** free base by portioning compound of formula iso-**VII**.chiral acid between an alkaline aqueous layer and a water-immiscible organic phase; activation of the hemi-acetal functionality by transforming the OH-group into an activated derivative; reaction of the resulting activated acetal with alcohol of formula **VIII.** Removal of impurities by extraction to get a solution of compound iso-**IX;**
c) removal of the *N*-protecting group to give the amine of formula iso-**X**;
d) oxidation of the amine of formula iso-**X** to the corresponding cyclic imine of formula iso-**X** and isolation of the cyclic imine of formula iso-**XI**;
e) reduction of imine of formula iso-**XI** with a catalyst and $H_2$ or an $H_2$ equivalent; removal of the catalyst by filtration to get the key intermediate of formula iso-**III**;
f) alkylation of compound of formula iso-**III** to give a compound of formula iso-**I** or of formula iso-**II** directly or via e.g. protected intermediates;
g) optionally, conversion of a compound of formula iso-**I** to a compound of formula iso-**II** by phosphorylation or a phosphorylation - deprotection sequence.

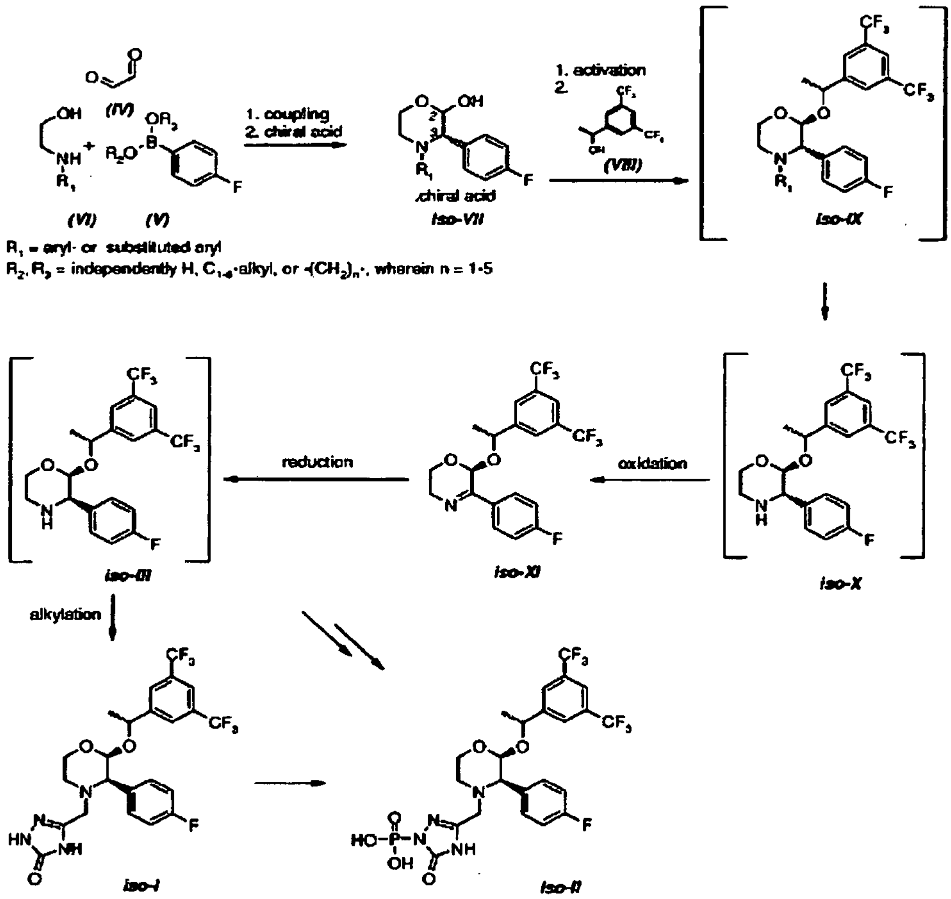


**18.** A compound of formula iso-**I**.

iso-I

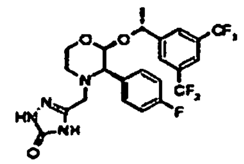

**19.** A compound of formula iso-**II**.

iso-II

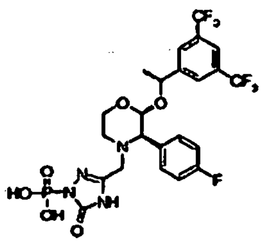

## Patentansprüche

1. Morpholinderivat der Formel VII oder der Formel iso-VII als Additionssalz mit einer chiralen Säure, wobei R1 Benzyl, substituiertes Benzyl oder eine andere Stickstoffschutzgruppe ist.

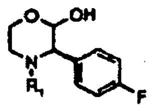

chirale Säure
VII

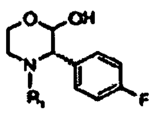

chirale Säure
iso-VII

2. Verbindungen nach Anspruch 1, wobei die chirale Säure ausgewählt ist aus der Gruppe, bestehend aus Weinsäure oder Weinsäurederivaten, Camphersulfonsäurederivaten wie 3-Bromcampher-10-sulfonsäure, Camphansäure, 10-Camphersulfon oder Camphorsäure, Aminosäuren wie Glutaminsäure, Valin oder Asparaginsäure, Mandelsäure oder Mandelsäurederivate wie a-Methoxy-$\alpha$-trifluormethylphenylessig- oder $\alpha$-Methoxyphenylessigsäure, Acet-oxy-5-etiensäure, Äpfelsäure, Menthyloxyessigsäure, N-($\alpha$-Methylbenzyl)-succinamidinsäure, N-[1-(1-Naphthyl)ethyl] succinaminsäure, N-(1-Phenylethyl)succinaminsäure, 1-Monomenthylphthalat, N,N-Bis[1-phenylethyl]-phthalaminsäure, N-(1-Phenylethyl)phthalaminsäure, 2-Phenylpropionsäure, Phenylcarbamoyloxypropionsäure, Pyroglutaminsäure, Chinasäure, 1,4-Benzo-dioxan-2-carbonsäure, 1,1'-Binaphthalen-2,2'-diyl-wasserstoffphosphat oder 5-Oxo-2-tetrahydrofurancarbonsäure in entweder enantiomerer oder diastereomerer Form.

3. Verbindungen nach Ansprüchen 1 - 2, wobei die chirale Säure Weinsäure oder ein Weinsäurederivat wie Di-O,O'-toluoylweinsäure, Di-O,O'-benzoylweinsäure, Di-O,O'-anisoylweinsäure oder O,O'-Dibenzoylweinsäuremono(dimethylamid) ist.

4. Verbindungen nach Ansprüchen 1 - 3, wobei das Weinsäurederivat Di-O,O'-toluoylweinsäure ist.

5. Verbindung nach Formel VII gemäß Anspruch 1, wobei die chirale Säure L-Di-O,O'-toluoylweinsäure ist und $R_1$ Benzyl ist.

6. Verbindung nach Formel iso-VII nach Anspruch 1, wobei die chirale Säure D-Di-O,O'-toluoylweinsäure ist und $R_1$ Benzyl ist.

7. Verfahren für die Herstellung von Morpholinderivaten gemäß Anspruch 1, umfassend die Schritte

   - Koppeln eines Aminoalkohols der Formel VI, 4-Fluorphenylborsäure oder eines $C_{1-6}$-Alkyl oder cyclischen Esters davon, und Glyoxal
   - Kristallisierung der erhaltenen Morpholinderivatsäure als Additionssalz mit einer chiralen Säure
   - Isolierung eines Produkts der Formel VII.chirale Säure oder der Formel iso-VII.chiraler Säure
   - gegebenenfalls Racemisieren des ungewünschten Isomers und erneutes Unterziehen des racemisierten Morpholinderivats einer Kristallisierung mit einer chiralen Säure.

8. Verfahren nach Anspruch 7, wobei die chirale Säure ausgewählt ist aus der Gruppe, bestehend aus Weinsäure oder Weinsäurederivaten, Camphersulfonsäurederivaten wie 3-Bromcampher-10-sulfonsäure, Camphansäure, 10-Camphersulfon- oder Camphansäure, Aminosäuren wie Glutaminsäure, Valin oder Asparaginsäure, Mandelsäure oder Mandelsäurederivaten wie a-Methoxy-a-trifluormethylphenylessig- oder a-Methoxyphenylessigsäure, Acetoxy-5-etiensäure, Äpfelsäure, Menthyloxyessigsäure, N-(a-Methylbenzyl)succin-amidinsäure, N-[1-(1-Naphthyl)ethyl] succinaminsäure, N-(1-Phenylethyl)-succinaminsäure, 1-Monomenthylphthalat, N,N-Bis[1-phenylethyl]phthalaminsäure, N-(1-Phenylethyl)phthalaminsäure, 2-Phenylpropionsäure, Phenylcarbamoyloxypropionsäure, Pyroglutaminsäure, Chinasäure, 1,4-Benzodioxan-2-carbonsäure, 1,1'-Binaphthalen-2,2'-diyl-wasserstoffphosphat oder 5-Oxo-2-tetrahydrofurancarbonsäure in entweder enantiomerer oder diastereomerer Form.

9. Verfahren nach Anspruch 7 - 8, wobei die chirale Säure Weinsäure oder ein Weinsäurederivat wie Di-O,O'-toluoylweinsäure, Di-O,O'-benzoylweinsäure, Di-O,O'-anisoylweinsäure oder O,O'-Dibenzoylweinsäuremono(dimethylamid) ist.

**10.** Verfahren nach Ansprüchen 7 - 9, wobei das Weinsäurederivat Di-O,O'-toluoylweinsäure ist.

**11.** Verfahren nach Anspruch 7, wobei das isolierte Produkt von Formel VII.L-Di-O,O'-toluoylweinsäure oder Formel iso-VII.D-Di-O.O'-toluoylweinsäure ist.

**12.** Verfahren nach Anspruch 11, wobei $R_1$ Benzyl oder substituiertes Benzyl ist.

**13.** Verwendung der Verbindung der Formel VII.L-Di-O,O'-toluoylweinsäure in der Synthese von Aprepitant oder Fosaprepitant.

**14.** Verwendung der Verbindung der Formel iso-VII.D-Di-O,O'-toluoylweinsäure in der Synthese von der Verbindungen von Formel iso-I oder iso-II.

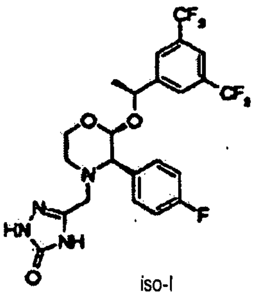
iso-I

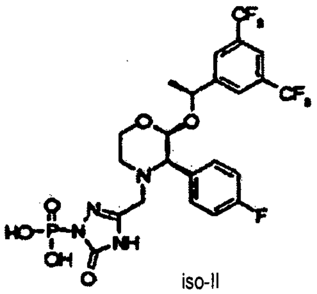
iso-II

**15.** Verfahren für die Herstellung von Aprepitant (I) oder Fosaprepitant (II), umfassend die Schritte

a) ein Dreikomponentenkoppeln eines Aminoalkohols der Formel VI, von 4-Fluorphenylborsäure oder eines $C_{1-6}$-Alkyl oder cyclischen Esters davon (Formel V), und von Glyoxal (IV) gefolgt von Kristallisierung des erhaltenen Morpholinderivats als Additionssalz mit einer chiralen Säure und Isolierung eines Produkts der Formel VII.chirale Säure;
b) Erzeugung von hemi-Acetal VII freier Base durch Portionieren von Verbindung der Formel VII.chirale Säure zwischen einer alkalischen wässrigen Schicht und einer nicht wassermischbaren organischen Phase; Aktivierung der hemi-Acetal-Funktionalität durch Transformieren der OH-Gruppe in ein aktiviertes Derivat; Reaktion des resultierenden aktivierten Acetals mit Alkohol der Formel VIII; Entfernung von Verunreinigungen durch Extraktion, um eine Lösung der Verbindung IX zu bekommen;
c) Entfernung der N-Schutzgruppe, um das Amin der Formel X zu geben;
d) Oxidation des Amins der Formel X zu dem entsprechenden cyclischen Imin der Formel X und Isolierung des cyclischen Imins der Formel XI;
e) Reduktion von Imin XI mit einem Katalysator und $H_2$ oder einem $H_2$-Äquivalent; Entfernung des Katalysators durch Filtration um das Schlüssel-Intermediat der Formel III zu geben;
f) Alkylierung der Verbindung der Formel III, um Aprepitant oder Fosaprepitant direkt oder via z. B. geschützten Intermediaten zu geben;
g) gegebenenfalls Umwandlung von Aprepitant zu Fosaprepitant durch Phosphorylierung oder einer Phosphorylierungs-Entschützungssequenz.

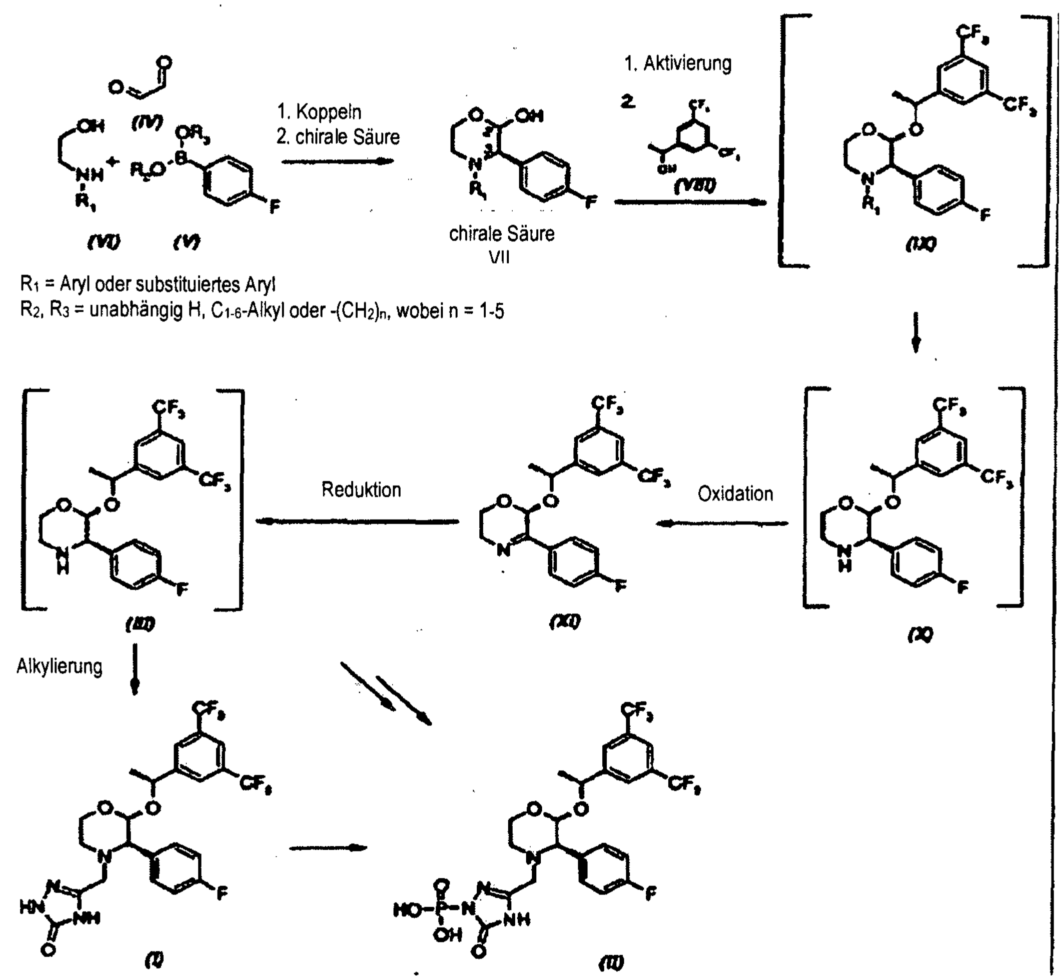


**16.** Verfahren nach Anspruch 15, **dadurch gekennzeichnet, dass** die isolierten Intermediate die Verbindungen der Formeln VII.chirale Säure und der Formel XI sind.

**17.** Verfahren für die Herstellung einer Verbindung von Formel iso-I oder iso-II umfassend die Schritte:

a) ein Dreikomponentenkoppeln eines Aminalkohols der Formel VI von 4-Fluorphenylborsäure oder eines $C_{1-6}$-Alkyl oder cyclischen Esters davon (Formel V) und von Glyoxal (IV), gefolgt durch Kristallisierung des erhaltenen Morpholinderivats als Additionssalz mit einer chiralen Säure und Isolierung des Produkts der Formel iso-VII.chirale Säure;
b) Erzeugung von hemi-Acetal iso-VII freie Base durch Portionieren der Verbindung von Formel iso-VII.chirale Säure zwischen einer alkalischen wässrigen Schicht und einer nicht wassermischbaren organischen Phase; Aktivierung der hemi-Acetal-Funktionalität durch Transformieren der OH-Gruppe in ein aktiviertes Derivat, Reaktion des resultierenden aktivierten Acetals mit Alkohol der Formel VIII; Entfernung von Verunreinigungen durch Extraktion, um eine Lösung der Verbindung iso-IX zu bekommen;
c) Entfernung der N-Schutzgruppe, um das Amin der Formel iso-X zu ergeben;
d) Oxidation des Amins der Formel iso-X zu dem entsprechenden cyclischen Imin der Formel iso-X und Isolierung des cyclischen Imins der Formel iso-XI;
e) Reduktion des Imins der Formel iso-XI mit einem Katalysator und $H_2$ oder einem $H_2$-Äquivalent; Entfernung des Katalysators durch Filtration, um das Schlüssel-Intermediat der Formel iso-III zu bekommen;
f) Alkylierung der Verbindung der Formel iso-III, um eine Verbindung der Formel iso-I oder der Formel iso-II direkt oder via z. B. geschützter Intermediate zu geben;
g) gegebenenfalls Umwandlung einer Verbindung der Formel iso-I zu einer Verbindung der Formel iso-II durch

Phosphorylierung oder einer Phosphorylierungs-Entschützungssequenz.

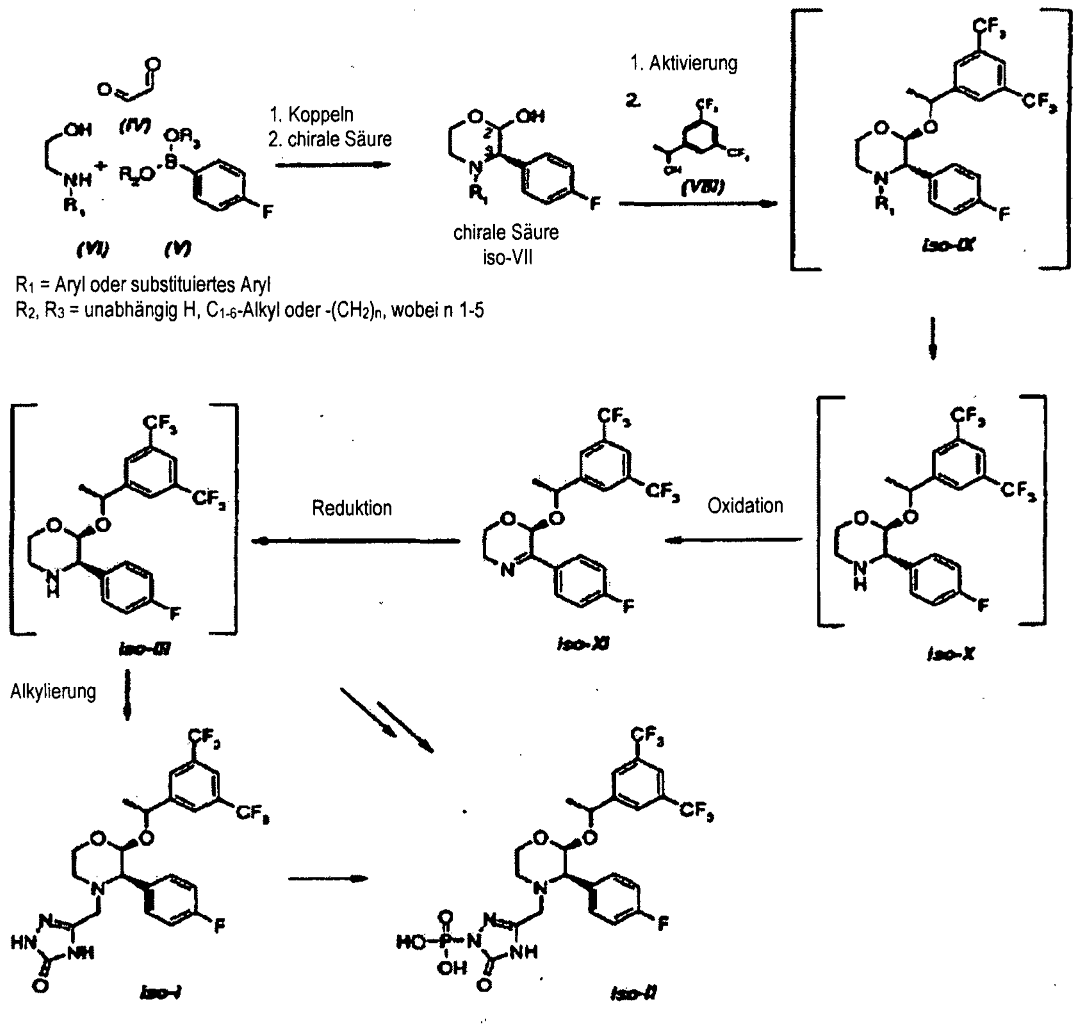


**18.** Verbindung der Formel iso-I

iso-I 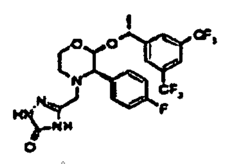

**19.** Verbindung der Formel iso-II

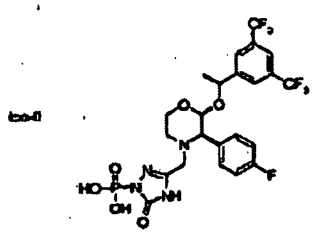

## Revendications

1. Dérivé morpholine de formule VII ou de formule iso-VII sous la forme d'un sel d'addition avec un acide chiral, dans lequel R1 est un benzyle, un benzyle substitué ou un autre groupe protecteur d'azote.

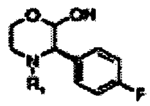

acide chiral
VII

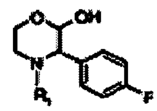

acide chiral
iso-VII

2. Composés selon la revendication 1, dans lesquels l'acide chiral est choisi dans le groupe constitué par l'acide tartrique ou les dérivés de l'acide tartrique, les dérivés de l'acide camphosulfonique tels que l'acide 3-bromocampho-10-sulfonique, l'acide camphanique, l'acide 10-camphosulfonique, ou l'acide camphorique, les acides aminés tels que l'acide glutamique, la valine, ou l'acide aspartique, l'acide mandélique ou les dérivés de l'acide mandélique tels que l'acide $\alpha$-méthoxy-$\alpha$-trifluorométhylphénylacétique ou $\alpha$-méthoxyphénylacétique, l'acide acétoxy-5-étiénique, l'acide malique, l'acide menthyloxyacétique, l'acide *N*-($\alpha$-méthylbenzyl)succinamidique, l'acide *N*-[1-(1-naphtyl)éthyl]succinamique, l'acide *N*-(1-phényléthyl)succinamique, le phtalate de 1-mono-menthyle, l'acide *N*,*N*-bis[1-phényléthyl]phtalamique, l'acide *N*-(1-phényléthyl)phtalamique, l'acide 2-phénylpropionique, l'acide phénylcarbamoyloxypropionique, l'acide pyroglutamique, l'acide quinique, l'acide 1,4-benzodioxane-2-carboxylique, l'hydrogénophosphate de 1,1'-binaphtalène-2,2'-diyle, ou l'acide 5-oxo-2-tétrahydrofurancarboxylique sous forme énantiomérique ou diastéréoisomérique.

3. Composés selon les revendications 1 à 2, dans lesquels l'acide chiral est l'acide tartrique ou un dérivé de l'acide tartrique tel que l'acide di-O,O'-toluoyl tartrique, l'acide di-O,O'-benzoyl tartrique, l'acide di-O,O'-anisoyl tartrique, ou le mono(diméthylamide) d'acide O.O'-dibenzoyl tartrique.

4. Composés selon les revendications 1 à 3, dans lesquels le dérivé d'acide tartrique est l'acide di-O,O'-toluoyl tartrique.

5. Composé de formule **VII** selon la revendication 1, dans lequel l'acide chiral est l'acide L-di-O.O'-toluoyl tartrique et $R_1$ est un benzyle.

6. Composé de formule ***iso*-VII** selon la revendication 1, dans lequel l'acide chiral est l'acide *D*-di-O,O'-toluoyl tartrique et $R_1$ est un benzyle.

7. Procédé pour la préparation de dérivés morpholine selon la revendication 1, comprenant les étapes consistant en

   - le couplage d'un amino-alcool de formule VI, d'acide 4-fluorophénylboronique ou d'un ester d'alkyle en $C_{1-6}$ ou cyclique de celui-ci, et de glyoxal
   - la cristallisation de l'acide dérivé morpholine obtenu sous la forme d'un sel d'addition avec un acide chiral
   - l'isolement d'un produit de formule **VII**.acide chiral ou de formule iso-**VII.**acide chiral
   - éventuellement la racémisation de l'isomère non souhaité et la soumission de nouveau du dérivé morpholine racémisé à une cristallisation avec un acide chiral.

**8.** Procédé selon la revendication 7, dans lequel l'acide chiral est choisi dans le groupe constitué par l'acide tartrique ou les dérivés de l'acide tartrique, les dérivés de l'acide camphosulfonique tels que l'acide 3-bromocampho-10-sulfonique, l'acide camphanique, l'acide 10-camphosulfonique, ou camphorique, les acides aminés tels que l'acide glutamique, la valine, ou l'acide aspartique, l'acide mandélique ou les dérivés de l'acide mandélique tels que l'acide $\alpha$-méthoxy-$\alpha$-trifluorométhylphénylacétique ou $\alpha$-méthoxyphénylacétique, l'acide acétoxy-5-étiénique, l'acide malique, l'acide menthyloxyacétique, l'acide *N*-($\alpha$-méthylbenzyl)succinamidique, l'acide *N*-[1-(1-naphtyl)éthyl]succinamique, l'acide *N*-(1-phényléthyl)succinamique, le phtalate de 1-mono-menthyle, l'acide *N*,*N*-bis[1-phényléthyl]phtalamique, l'acide *N*-(1-phényléthyl)phtalamique, l'acide 2-phénylpropionique, l'acide phénylcarbamoyloxypropionique, l'acide pyroglutamique, l'acide quinique, l'acide 1,4-benzodioxane-2-carboxylique, l'hydrogénophosphate de 1,1'-binaphtalène-2,2'-diyle, ou l'acide 5-oxo-2-tétrahydrofurancarboxylique sous forme énantiomérique ou diastéréoisomérique.

**9.** Procédé selon la revendication 7 à 8, dans lequel l'acide chiral de l'acide tartrique ou un dérivé de l'acide tartrique tel que l'acide di-O,O'-toluoyl tartrique, l'acide di-O,O'-benzoyl tartrique, l'acide di-O,O'-anisoyl tartrique, ou le mono(diméthylamide) d'acide O,O'-dibenzoyl tartrique.

**10.** Procédé selon les revendications 7 à 9, dans lequel le dérivé d'acide tartrique est l'acide di-O,O'-toluoyl tartrique.

**11.** Procédé selon la revendication 7, dans lequel le produit isolé est de formule **VII**.acide *L*-di-O,O'-toluoyl tartrique ou de formule iso-**VII**.acide *D*-di-O,O'toluoyl tartrique.

**12.** Procédé selon la revendication 11, dans lequel $R_1$ est un benzyle ou un benzyle substitué.

**13.** Utilisation du composé de formule **VII**.acide L-di-O,O'-toluoyl tartrique dans la synthèse de l'aprépitant ou du fosaprépitant.

**14.** Utilisation du composé de formule iso-**VII**.acide *D*-di-O,O'toluoyl tartrique dans la synthèse des composés de formule **iso-I** ou **iso-II.**

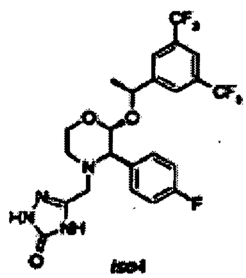
iso-I

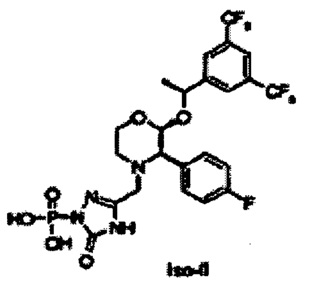
iso-II

**15.** Procédé pour la préparation de l'aprépitant (**I**) ou du fosaprépitant (**II**), comprenant les étapes consistant en

a) un couplage à trois composants d'un amino-alcool de formule **VI**, d'acide 4-fluorophénylboronique ou d'un ester d'alkyle en $C_{1-6}$ ou cyclique de celui-ci (formule **V**), et de glyoxal (**IV**) suivi d'une cristallisation du dérivé morpholine obtenu sous la forme d'un sel d'addition avec un acide chiral et de l'isolement d'un produit de formule **VII**.acide chiral ;
b) la génération d'une base libre hémi-acétal **VII** par fractionnement d'un composé de formule **VII**.acide chiral entre une couche aqueuse alcaline et une phase organique non miscible à l'eau ; l'activation de la fonctionnalité hémi-acétal par transformation du groupe OH en un dérivé activé ; la réaction de l'acétal activé résultant avec un alcool de formule **VIII.** L'élimination des impuretés par extraction pour obtenir une solution du composé **IX** ;
c) l'élimination du groupe N-protecteur pour donner l'amine de formule **X** ;
d) l'oxydation de l'amine de formule **X** en l'imine cyclique correspondante de formule **X** et l'isolement de l'imine cyclique de formule **XI** ;
e) la réduction de l'imine **XI** avec un catalyseur et de l'$H_2$ ou un équivalent de $H_2$ ; l'élimination du catalyseur par filtration, pour obtenir l'intermédiaire clé de formule **III** ;
f) l'alkylation du composé de formule **III** pour donner de l'aprépitant ou du fosaprépitant directement ou *via* des intermédiaires protégés par exemple ;

g) éventuellement, la conversion de l'aprépitant en fosaprépitant par phosphorylation ou une séquence de phosphorylation - déprotection.

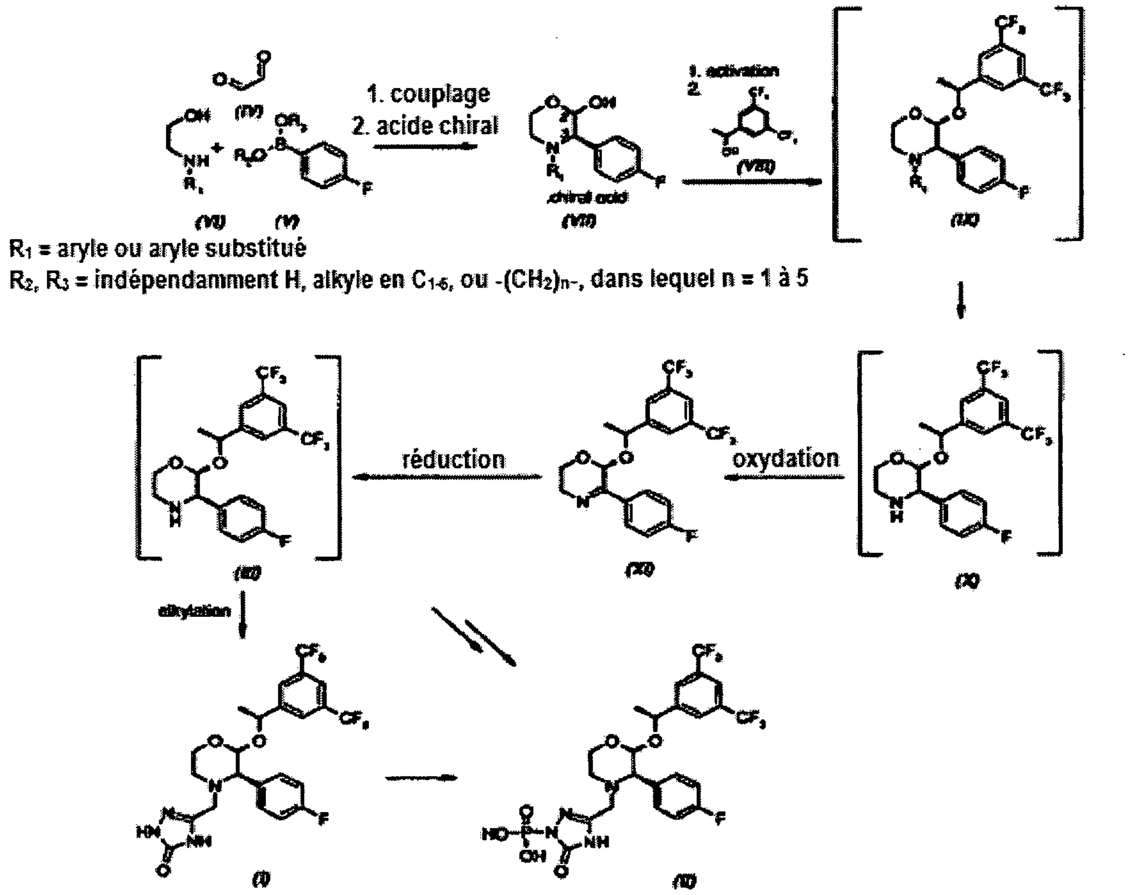


**16.** Procédé selon la revendication 15, **caractérisé en ce que** les intermédiaires isolés sont les composés de formule **VII**.acide chiral et de formule **XI**.

**17.** Procédé pour la préparation d'un composé de formule **iso-I** ou **iso-II**, comprenant les étapes consistant à

a) un couplage à trois composants d'un amino-alcool de formule **VI,** d'acide 4-fluorophénylboronique ou d'un ester d'alkyle en $C_{1-6}$ ou cyclique de celui-ci (formule **V**), et de glyoxal (**IV**) suivi d'une cristallisation du dérivé morpholine obtenu sous la forme d'un sel d'addition avec un acide chiral et de l'isolement d'un produit de formule iso-**VII**.acide chiral ;
b) la génération d'une base libre hémi-acétal iso-**VII** par fractionnement d'un composé de formule iso-**VII**.acide chiral entre une couche aqueuse alcaline et une phase organique non miscible à l'eau ; l'activation de la fonctionnalité hémi-acétal par transformation du groupe OH en un dérivé activé ; la réaction de l'acétal activé résultant avec un alcool de formule **VIII.** L'élimination des impuretés par extraction pour obtenir une solution du composé iso-**IX** ;
c) l'élimination du groupe *N*-protecteur pour donner l'amine de formule iso-**X** ;
d) l'oxydation de l'amine de formule iso-**X** en l'imine cyclique correspondante de formule iso-**X** et l'isolement de l'imine cyclique de formule iso-**XI** ;
e) la réduction de l'imine de formule iso-**XI** avec un catalyseur et de l'$H_2$ ou un équivalent de $H_2$ ; l'élimination du catalyseur par filtration, pour obtenir l'intermédiaire clé de formule iso-**III** ;
f) l'alkylation du composé de formule iso-**III** pour donner un composé de formule iso-**I** ou de formule iso-**II** directement ou *via* des intermédiaires protégés par exemple ;
g) éventuellement, la conversion d'un composé de formule iso-**I** ou d'un composé de formule iso-**II** par phosphorylation ou une séquence de phosphorylation-déprotection.

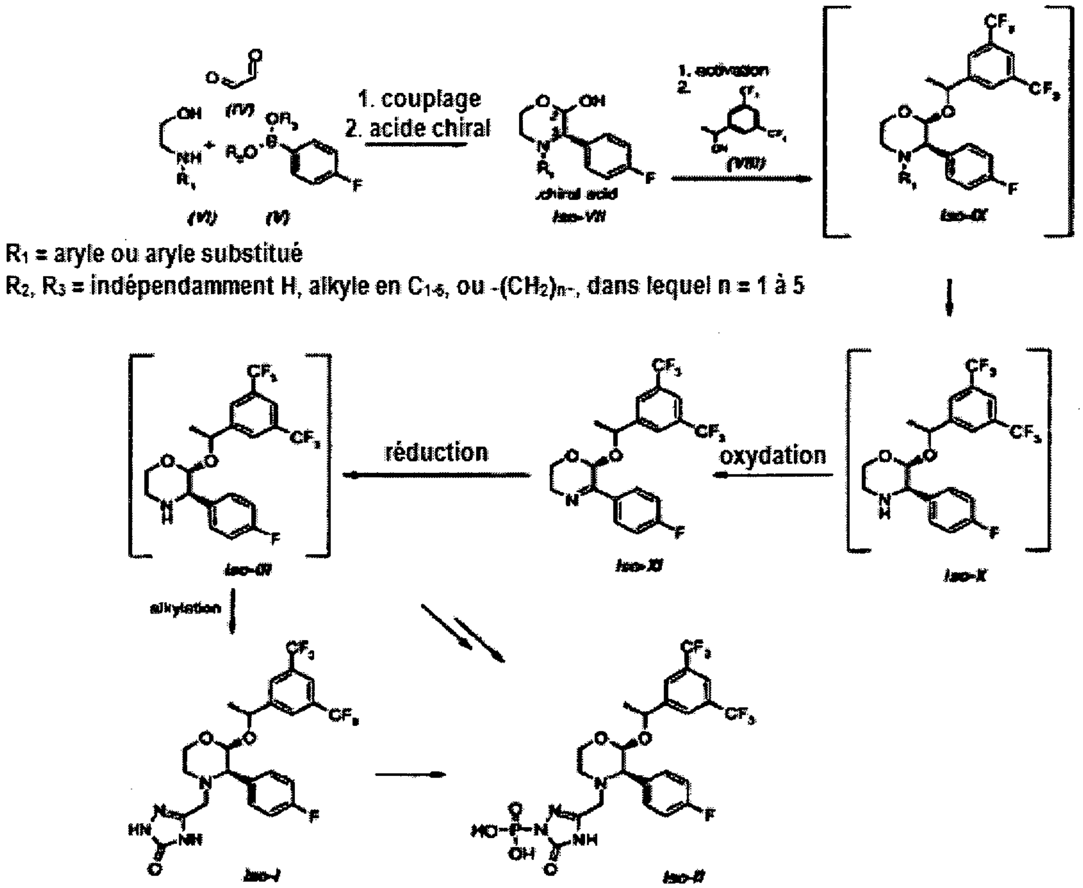


18. Composé de formule iso-I.

iso-I

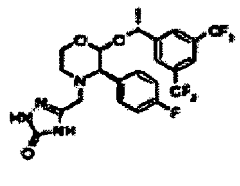

19. Composé de formule iso-II

iso-II

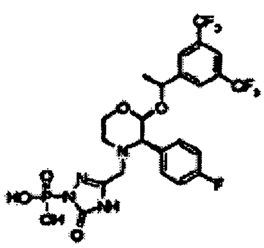

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- EP 0734381 B1
- EP 0748320 B1
- WO 99065900 A1
- WO 2003089429 A1
- WO 2001096319 A1
- WO 2001096320 A1
- US 5668280 A
- US 6130331 A
- WO 2001094322 A1
- WO 2001094323 A1
- WO 2001094324 A1
- WO 2001096315 A1
- WO 2006060110 A1


### Non-patent literature cited in the description


- *Chemistry- A european Journal,* 2002, vol. 8 (6), 1372-76
- **THEODORA W. GREENE ; PETER G. M. WUTS.** Protecting Groups in Organic Syntheses. John Wiley & Sons, 1999
- **ZHAO, M. M. ; MCNAMARA, J. M. ; HO, G.-J. ; EMERSON, K. M. ; SONG, Z. J. ; TSCHAEN, D.M. ; BRANDS, K. M. J ; DOLLING, U.-H. ; GRABOWSKI, E. J. J. ; REIDER, P.J.** *J. Org. Chem.,* 2002, vol. 67, 6743-6747
- **ZHAO, M. M. ; MCNAMARA, J. M. ; HO, G.-J. ; EMERSON, K. M. ; SONG, Z. J. ; TSCHAEN, D.M. ; BRANDS, K. M. J. ; DOLLING, U.-H. ; GRABOWSKI, E. J. J. ; REIDER, P.J.** *J. Org. Chem.,* 2002, vol. 67, 6743-6747
- **ZHAO, M. M. ; MCNAMARA, J. M. ; HO, G.-J. ; EMERSON, K. M. ; SONG, Z. J. ; TSCHAEN, D. M. ; BRANDS, K. M. J. ; DOLLING, U.-H. ; GRABOWSKI, E. J. J. ; REIDER, P.J.** *J. Org. Chem.,* 2002, vol. 67, 6743-6747